# EUROPEAN PATENT SPECIFICATION

(11) **EP 2 500 409 B1**
(45) Date of publication and mention of the grant of the patent: **07.01.2015**
(21) Application number: 09851235.3
(22) Date of filing: 11.11.2009
(51) Int. Cl.: C12M 1/00

(54) **DEVICE FOR MODULATING PGC-1 EXPRESSION**
VORRICHTUNG ZUR MODULATION DER PGC-1-EXPRESSION
DISPOSITIF PERMETTANT DE MODULER L'EXPRESSION DE PGC-1

(43) Date of publication of application: 19.09.2012
(73) Proprietor: Nippon Sigmax Co., Ltd., Tokyo 163-6033 (JP); Hiroshima University, Hiroshima 739-8511 (JP)
(72) Inventor: HIGASHI, Yukihito, Hiroshima-shi, Hiroshima 734-8551 (JP); KUBO, Hiroshi, Shinjuku-ku Tokyo 163-6033 (JP); SUMIDA, Kiyoshi, Shinjuku-ku Tokyo 163-6033 (JP); NISHIMURA, Yoshihiro, Shinjuku-ku Tokyo 163-6033 (JP); NIIMI, Nobuo, Shinjuku-ku Tokyo 163-6033 (JP)
(74) Representative: Cabinet Plasseraud
(86) International application number: PCT/JP2009/006025
(87) International publication number: WO 2011/058600

(56) References cited:
- WO-A1-2010/009141
- JP-A- 2005 304 918
- JP-A- 2005 304 918
- US-A- 4 530 360
- US-A1- 2003 153 849
- US-A1- 2003 225 331
- US-A1- 2006 241 522
- US-A1- 2008 255 049
- US-B1- 7 429 249
- WANG: "Nitric oxide mediates ultrasound-induced hypoxia-inducible factor-1alpha activation and vascular endothelial growth factor-A expression in human osteoblasts", BONE (NEW YORK), vol. 35, no. 1, 1 July 2004 (2004-07-01), pages 114-123, XP055056414,
- ARANY Z. ET AL.: 'HIF-independent regulation of VEGF and angiogenesis by the transcriptional coactivator PGC-1alpha' NATURE vol. 451, no. 7181, 2008, pages 1008 - 1013
- FRAISL P. ET AL.: 'Hungry for Blood Vessels: Linking Metabolism and Angiogenesis' DEV. CELL vol. 14, no. 3, 2008, pages 313 - 314
- HIDAKA T. ET AL.: 'Low-intensity Pulsed Ultrasound (LIPUS) Induces Angiogenesis in a Rat Ischemic Hind Limb Model: Role of VEGF' CIRC. J. vol. 72, no. SUPPL., 2008, page 247
- 'Dai 23 Kai Annual Research Meeting of the Japanese Orthopaedic Association Abstracs', vol. 82, 2008 article ATSUKO OKUBO ET AL.: 'Tei Shutsuryoku Choonpa Pulse Shosha ni yoru Hito Kotsuzui Kan'yokei Kansaibo no Dlx Idenshi Kyo Hatsugen to Hone Bunka Sokushin', page S1195
- POUNDER N M ET AL: "Low intensity pulsed ultrasound for fracture healing: A review of the clinical evidence and the associated biological mechanism of action", ULTRASONICS, IPC SCIENCE AND TECHNOLOGY PRESS LTD. GUILDFORD, GB, vol. 48, no. 4, 1 August 2008 (2008-08-01) , pages 330-338, XP023439273, ISSN: 0041-624X, DOI: 10.1016/J.ULTRAS.2008.02.005 [retrieved on 2008-03-27]

## Description

### Technical Field

The present invention relates to a device for modulating PGC-1 expression, preferably suitable for treating ischemic disease.

### Background Art

Examples of Ischemic diseases caused by decreased blood flow include angina pectoris, myocardial infarction, cerebral infarction, arteriosclerosis obliterans, chronic arteriosclerosis obliterans, and Burger's disease. Arteriosclerosis obliterans (ASO) is a disease caused by chronic clogging mainly in large blood vessels of the lower extremities. ASO causes cold feeling in a mild case, but may cause necrosis of an extremity in a severe case. Burger's disease, which can be called "thromboangiitis obliterans (TAO)", is a disease that results in obstructive vasculitis in major arteries in the extremities.

Treatments for ASO and TAO vary depending on the stage of progression. For a mild case, anti-thrombotic medicine, fish oil, prostacyclin, and/or the like is used internally. Further, prostaglandin E1, antithrombin agent, and/or the like is administered intravenously. In an advanced stage of ASO and TAO, an endovascular treatment, such as vascular bypass procedure, balloon dilatation, and/or stent placement, is surgically provided.

Other than these treatment methods, a revascularization treatment can be provided. In the revascularization treatment, cells, physiologically active substance, scaffolding, or others is transplanted into an affected site. This brings regeneration of defective or dysfunctional vessels to improve the flow of blood. In a cell therapy that would stimulate angiogenesis, bone-marrow cells, peripheral blood stem cell (PBSCT: Peripheral blood stem cell transplantation), peripheral mononuclear cells are infused into several tens of spots of an affected site. Still further, a gene therapy is provided. In the gene therapy, genes (HGF, VEGF, etc.) that express angiogenesis-inducing hormone are administered intramuscularly to induce angiogenesis.

Further, Patent Literature 1 describes another angiogenesis treatment method, which is a technique of subjecting an affected site to ultrasonic irradiation to promote angiogenesis in tissues of the affected site. An ultrasonic treatment has long been used for many diseases with the aim of obtaining a treatment effect that would be caused by improvement of blood flow through hyperthermic effect. For example, Patent Literature 2 describes a technique of using ultrasonic pulse for the purpose of accelerating the healing of bone fractures.

### Citation List

### Patent Literatures

### Patent Literature 1

Japanese Unexamined Patent Application Publication, Tokuhyo, No. 2005-523132 (Publication D ate: August 4, 2005)

### Patent Literature 2

Specification of U.S. Patent No. 4530360 (Publication Date: July 23, 1985)

### Non-patent Literatures

### Non-patent Literature 1

Zoltan Arany et al., nature, Vol 451(21), p1008-1012, February 2008

### Non-patent Literature 2

Jiandie Lin et al., nature, Vol 418(15), p797-801, August 2002

A further ultrasound device is disclosed in US 7,429,249 B1.

### Summary of Invention

### Technical Problem

When the ischemic disease progresses, the above-described treatment methods are used. However, these treatment methods have the following problems. The surgical treatment associated with open surgery has various risks such as risk of infection and imposes a great physical burden on patients. Furthermore, a treatment with a cell transplantation into an affected site requires removal of a large amount of bone marrow and blood and therefore becomes a great physical burden on patients. Still further, the transplantation of physiologically active substance or scaffolding becomes a great economic burden because of the expensiveness of preparation of a material for transplantation. Furthermore, such transplantation is questionable in view of safety and stability. Moreover, the gene therapy is questionable in view of safety and stability and is applied only to a patient in serious condition.

In addition, the treatment as described in Patent Literature 1 using ultrasonic waves has the following problems. Because of its unraveled mechanism of angiogenesis promotion, it is impossible to optimize conditions appropriate to a patient and his/her disease status, thereby making it difficult to provide an appropriate treatment to the patient. Besides, for the same reason, the treatment using ultrasonic waves is unclear in its effectiveness.

The present invention has been made to solve the above-described problems, and an object thereof is to provide a device for modulating PGC-1 expression to enable a treatment for ischemic disease by promoting angiogenesis in a living body without an excessive burden on a patient.

### Solution to Problem

The invention is defined in the appended set of claims.

Various researches on the mechanism of angiogenesis have been conducted so far, and it is known that a vascular endothelial growth factor (VEGF) used for the above-described gene therapy is involved in angiogenesis. In addition, transcription of VEGF is modulated by its transcription factor, HIF (Hypoxia inducible factor), or its transcription factor coactivator, PGC-1 (Peroxisome proliferator activated receptor γ coactivator).

Non-patent Literature 1 describes that PGC-1α, without depending on HIF, modulates VEGF and VEGF-triggered angiogenesis, and that transcription of PGC-1α is promoted by exercise. It is known that PGC-1 exists in various kinds of tissues such as a brown adipose tissue and a skeletal muscle tissue, and that PGC-1 activates mitochondrial biogenesis and oxidative metabolism (Non-patent Literature 2).

The inventors of the present invention diligently worked from their own points of view and accomplished the present invention by finding that application of physical and mechanical stimulus to a living body by ultrasonic irradiation causes promotion of PGC-1 expression and by realizing a treatment using the angiogenesis promotion through PGC-1-VEGF cascade.

That is, a device for modulating PGC-1 expression according to the present invention includes ultrasonic irradiation means for irradiating a subject with ultrasonic waves. Furthermore, a system for treating ischemic disease according to the present invention includes the device for modulating PGC-1 expression.

The device for modulating PGC-1 expression according to the present invention includes ultrasonic irradiation means for irradiating a subject living body with ultrasonic waves. This allows modulating PGC-1 expression in the living body. Thus, it is possible to control PGC-1-associated biological reaction and to control a factor, like VEGF, that is downstream of PGC-1. Therefore, it is possible to modulate PGC-1 expression by ultrasonic irradiation of a living body to modulate VEGF expression, thus promoting VEGF-associated angiogenesis.

Thus, according to the device for modulating PGC-1 expression according to the present invention for treating ischemic disease , a patient who could use the treatment is not limited. This is because the cost of the treatment is low, safety of the treatment is established, and the treatment involves a high level of safety.

Further, the device for modulating PGC-1 expression according to the present invention includes controlling means for controlling the ultrasonic irradiation means to irradiate the living body with ultrasonic waves of a frequency in a range from 0.5 MHz to 3 MHz ± 10 %. Still further, in the device for modulating PGC-1 expression according to the present invention, the controlling means controls the ultrasonic irradiation means to irradiate the living body with ultrasonic waves for a period of time in a range from 20 minutes to 40 minutes ± 10 % per day. With the above arrangement, it is possible to promote PGC-1 expression more effectively.

Yet further, in the device for modulating PGC-1 expression according to the present invention, it is preferable that the ultrasonic irradiation means further includes at least one ultrasonic emission terminal that non-invasively contacts the living body via a medium and emits the ultrasonic waves for irradiation of the living body. This makes it possible to non-invasively modulate PGC-1 expression, thus promoting angiogenesis.

Further, the device for modulating PGC-1 expression according to the present invention preferably includes controlling means for controlling the ultrasonic irradiation means so that a plurality of the ultrasonic emission terminals are sequentially driven by time division to emit the ultrasonic waves. This makes it possible to efficiently irradiate the living body with ultrasonic waves without concentration of ultrasonic energy onto a particular spot of the living body.

### Advantageous Effects of Invention

The device for modulating PGC-1 expression according to the present invention includes ultrasonic irradiation means for irradiating a subject living body with ultrasonic waves. This makes it possible to modulate PGC-1 expression in the living body, thus bringing about the effect of promoting angiogenesis in which VEGF is involved.

### Brief Description of Drawings

Fig. 1 is a block diagram showing an embodiment of a device for modulating PGC-1 expression according to the present invention.
Fig. 2 is a block diagram showing other embodiment of a device for modulating PGC-1 expression according to the present invention.
Fig. 3 (a) and (b) are schematic diagrams showing examples of the configuration of a transducer provided in a PGC-1 expression-modulating device 1 according to the present invention.
Fig. 4 is a graph showing the result of an experiment aimed at investigating changes in PGC-1α expression level by ultrasonic irradiation.
Fig. 5 is a graph showing the result of an experiment aimed at investigating changes in VEGF-A expression level by ultrasonic irradiation.
Fig. 6 is a graph showing the result of an experiment aimed at investigating changes in VEGF-B expression level by ultrasonic irradiation.
Fig. 7 is a graph showing the result of an experiment aimed at investigating changes in VEGF-C expression level by ultrasonic irradiation.
Fig. 8 is a graph showing the result of an experiment aimed at investigating changes in HIF-1α expression level by ultrasonic irradiation.
Fig. 9 is a laser Doppler image photograph obtained for investigating the influence of ultrasonic irradiation on restoration of blood flow.
Fig. 10 is a laser Doppler image photograph obtained for investigating the influence of ultrasonic irradiation on restoration of blood flow.
Fig. 11 is a laser Doppler image photograph obtained for investigating the influence of ultrasonic irradiation on restoration of blood flow.
Fig. 12 is a laser Doppler image photograph obtained for investigating the influence of ultrasonic irradiation on restoration of blood flow.
Fig. 13 is a laser Doppler image photograph obtained for investigating the influence of ultrasonic irradiation on restoration of blood flow.
Fig. 14 is a laser Doppler image photograph obtained for investigating the influence of ultrasonic irradiation on restoration of blood flow.
Fig. 15 is a micrograph showing the result of hematoxylin-eosin staining in an investigation of the influence of ultrasonic irradiation on angiogenesis.
Fig. 16 is a micrograph showing the result of hematoxylin-eosin staining in an investigation of the influence of ultrasonic irradiation on angiogenesis.
Fig. 17 is a micrograph showing the result of hematoxylin-eosin staining in an investigation of the influence of ultrasonic irradiation on angiogenesis.
Fig. 18 is a micrograph showing the result of alkaline phosphatase staining in an investigation of the influence of ultrasonic irradiation on angiogenesis.
Fig. 19 is a micrograph showing the result of alkaline phosphatase staining in an investigation of the influence of ultrasonic irradiation on angiogenesis.
Fig. 20 is a micrograph showing the result of alkaline phosphatase staining in an investigation of the influence of ultrasonic irradiation on angiogenesis.
Fig. 21 is a graph showing the result of alkaline phosphatase staining in an investigation of the influence of ultrasonic irradiation on a capillary density ratio.
Fig. 22 is a micrograph showing the experimental result of immunohistochemical staining in an investigation of the influence of ultrasonic irradiation on PGC-1α expression.
Fig. 23 is a micrograph showing the result of immunohistochemical staining in an investigation of the influence of ultrasonic irradiation on VEGF-A expression.
Fig. 24 is a micrograph showing the result of immunohistochemical staining in an investigation of the influence of ultrasonic irradiation on VEGF-B expression.
Fig. 25 is a micrograph showing the result of immunohistochemical staining in an investigation of the influence of ultrasonic irradiation on VEGF-C expression.
Fig. 26 is a micrograph showing the result of immunohistochemical staining using a rabbit negative control antibody.
Fig. 27 is a micrograph showing the result of immunohistochemical staining using a goat negative control antibody.
Fig. 28 is a micrograph showing the result of immunohistochemical staining using a CD31 antibody.
Fig. 29 is a micrograph showing the result of immunohistochemical staining using a CD31 antibody.
Fig. 30 is a micrograph showing the result of immunohistochemical staining using a CD31 antibody.
Fig. 31 is a graph showing the result of immunohistochemical staining using a CD31 antibody in an investigation of the influence of ultrasonic irradiation on a capillary density ratio.

### Description of Embodiments

### [PGC-1 expression modulating device]

### (First Embodiment)

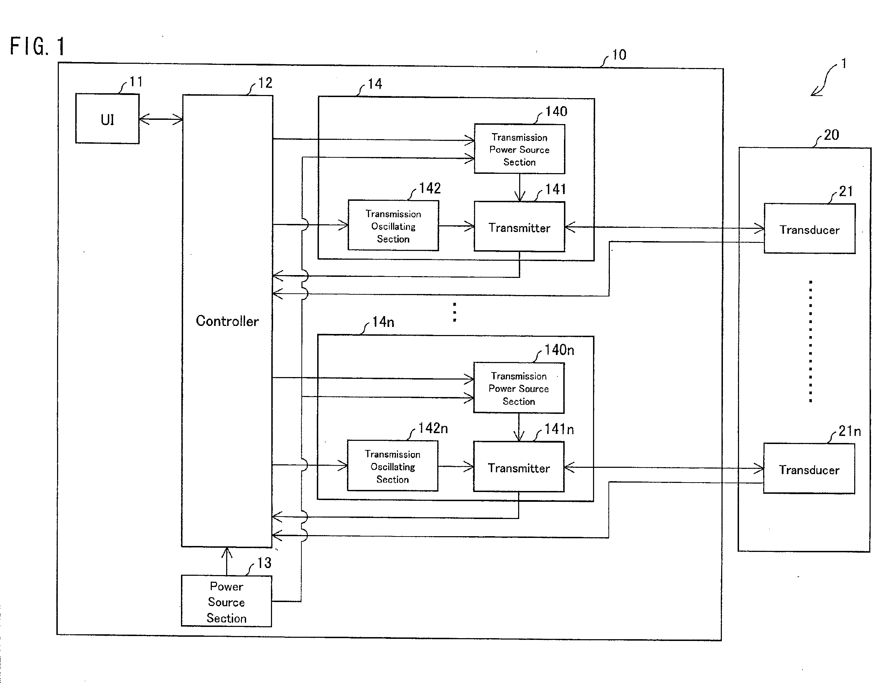

With reference to Fig. 1, the following will describe one embodiment of a PGC-1 expression-modulating device according to the present invention. Fig. 1 is a block diagram showing one embodiment of a PGC-1 expression-modulating device 1 according to the present invention. As shown in Fig. 1, the PGC-1 expression-modulating device 1 according to the present invention is constituted by a main unit 10 and a probe 20. The main unit 10 includes a user interface (UI) 11, a controller (controlling means) 12, a power source section 13, and at least one ultrasonic wave transmitting section 14 (ultrasonic irradiation means). The probe 20 is provided with at least one transducer (ultrasonic emission terminal) 21. The probe 20 may be provided in a housing independent from a housing where the main unit 10 is provided. Alternatively, the probe 20 may be provided in the same housing as that of the main unit 10 in such a manner that the probe 20 is independent from the main unit 10. Further, the probe 20 may be connected to the main unit 10 via a cable or the like so that the probe 20 can operate at a location away from the main unit 20.

As shown in Fig. 1, the main unit 10 includes n ultrasonic wave transmitting sections (ultrasonic wave transmitting sections 14 through 14n). Moreover, n transducers (transducers 21 through 21n) are provided in the probe 20 so as to be in a one-to-one correspondence with the ultrasonic wave transmitting sections. Note that "n" indicates an integer that is 1 or more and can be arbitrarily selected. The transducers 21 through 21n are connected to the ultrasonic wave transmitting sections 14 through 14n, respectively.

Ultrasonic waves having been transmitted from the ultrasonic wave transmitting sections 14 through 14n are emitted from the transducers 21 through 21n, which are connected to the ultrasonic wave transmitting sections 14 through 14n, and a living body as a subject of irradiation is then irradiated with the ultrasonic waves. Note that the ultrasonic wave transmitting sections 14 through 14n are all configured in a similar manner. Also, the transducers 21 through 21n are all configured in a similar manner. For this reason, the following will mainly describe the ultrasonic wave transmitting section 14 and the transducer 21.

The user interface 11 may be provided with (a) a display section configured to display a state and the like of the PGC-1 expression-modulating device 1 and (b) an input section that accepts an entry from a user. When the user interface 11 accepts an entry from a user, the user interface 11 transmits an input signal, which indicates the entry, to the controller 12, and the controller 12 then controls the constituent elements in accordance with the received input signal.

Further, the user interface 11 receives a signal that has been transmitted from the controller 12 and indicates the state of the PGC-1 expression-modulating device 1, and the user interface 11 then causes the display section to display the state of the PGC-1 expression-modulating device 1 in accordance with the received signal. The input section of the user interface 11 may be configured to be the one through which the user can enter, for example, a frequency of ultrasonic waves for irradiation, ultrasonic irradiation time, and the like.

The controller 12 controls the constituent elements of the PGC-1 expression-modulating device 1. Under the controller 12, the ultrasonic wave transmitting section 14 transmits ultrasonic waves to the transducer 21 provided in the probe 20 so that the transducer 21 emits the ultrasonic waves to a living body. Specifically, the controller 12 causes a transmission oscillating section 142 of the ultrasonic wave transmitting section 14 to output ultrasonic waves and then causes a transmitter 141 of the ultrasonic wave transmitting section 14 to transmit the ultrasonic waves to the transducer 21. Further, the controller 12 controls transmission of ultrasonic waves from the ultrasonic wave transmitting section 14 in accordance with the user's entry into the user interface 11, and the controller 12 controls the user interface 11 to display the state of the PGC-1 expression-modulating device 1 and the like. Further, the controller 12 supplies electric power fed from the power source section 13, to a transmission power source section 140.

Still further, the controller 12 controls ultrasonic wave output from the transmission oscillating section 142 so that the transducer 21 outputs ultrasonic waves of a specified value. Under control of the controller 12, the transducer 21 outputs ultrasonic waves of a set value that is suitably set to obtain a desired effect. Further, the controller 12 may be configured to control output of ultrasonic waves for the purpose of correcting variations in sensitivity of the transducer 21. Control of the ultrasonic wave output can be realized by control of an oscillation frequency of the transmission oscillating section 142, control of a transmission voltage at the transmission of ultrasonic waves from the transmitter 143 to the transducer 21, or the like control.

The power source section 13 supplies electric power to the constituent elements of the PGC-1 expression-modulating device 1. The power source section 13 supplies electric power provided by a commercial power source, a battery, or the like, to the controller 12 and the transmission power source section 140 of the ultrasonic wave transmitting section 14.

The ultrasonic wave transmitting section 14 includes the transmission power source section 140, the transmitter 141, and the transmission oscillating section 142. The transmission power source section 140 supplies electric power provided by the controller 12 or the power source section 13 to the transmitter 141. The transmitter 141 transmits ultrasonic waves outputted by the transmission oscillating section 142 to the transducer 21. The transmission oscillating section 142 oscillates ultrasonic waves in accordance with an instruction from the controller 12, and then outputs the ultrasonic waves to the transmitter 141. As the transmission power source section 140, the transmitter 141, and the transmission oscillating section 142, conventionally known power source section, transmitter, and ultrasonic wave oscillating section, and the like can be used.

The transducer 21 emits the ultrasonic waves, which have been transmitted by the transmitter 141 of the ultrasonic wave transmitting section 14, to a living body. For example, assume that the PGC-1 expression-modulating device 1 is used as a treating device for ASO of which an affected site normally extend in a large area. In this case, it is preferable that an area to be irradiated with ultrasonic waves is large. In the PGC-1 expression-modulating device 1 shown in Fig. 1, the probe 20 includes a plurality of transducers 21 through 21n. This enables ultrasonic irradiation over a larger area.

In this case, the plurality of transducers 21 through 21n are configured to be attached to a material having flexibility, e.g. silicon, by adhesion or the like method, so that the affected site can be covered with the material provided with the transducers 21 through 21n. The transducer 21 is provided in a housing of the probe 20 so that the transducer 21 can output ultrasonic waves for irradiation over a living body while being in non-invasive contact with the living body via a medium. Details of the configuration of the transducer 21 will be described later.

In order to output ultrasonic waves of a set value, to correct variations in sensitivity of the ultrasonic transducers, and to keep ultrasonic power constant, the PGC-1 expression-modulating device 1 may include a feedback mechanism that monitors transmission of the ultrasonic waves from the transmitter 141. With this arrangement, the controller 12 gets feedback on a state of transmission of the ultrasonic waves from the transmitter 141. In accordance with the feedback, the controller 12 controls ultrasonic wave output from the transmission oscillating section 142 and the transmitter 141.

In addition, for detection of failed coupling between the transducer 21 and the affected site, the PGC-1 expression-modulating device 1 may also include a feedback mechanism that monitors ultrasonic wave output from the transducer 21. With this arrangement, the controller 12 or the transmitter 141 gets feedback on a state of coupling between the transducer 21 and the affected site. In accordance with the feedback, the controller 12 controls ultrasonic wave output from the transmission oscillating section 142 and the transmitter 141.

As described above, the PGC-1 expression-modulating device 1 includes the ultrasonic wave transmitting section 14 that performs ultrasonic irradiation over a living body. This makes it possible to control PGC-1 expression in the affected site subjected to ultrasonic irradiation in the living body. PGC-1 is a transcription factor coactivator that centralizes a signaling pathway that regulates metabolism. PGC-1 plays a role in regulating mitochondrial oxidative metabolism and maintaining sugar, lipid, and energy homeostasis. Known as family members of PGC-1 are PGC-1α and PGC-1β. PGC-1α and PGC-1β share homology with each other.

When PGC-1 expression is promoted by the PGC-1 expression-modulating device 1 according to the present invention, the promotion of PGC-1 expression enhances the transcription of VEGF. VEGF is a factor involved in angiogenesis. According to the present invention, the promotion of PGC-1 expression enhances transcription of family members of VEGF, e.g. VEGF-A, VEGF-B, and VEGF-C.

It is known that enhancement of transcription of PGC-1α by ischemia and exercise induces enhancement of transcription of VEGF, which in turn promotes angiogenesis (Non-Patent Document 1). The inventors of the present invention have newly found that ultrasonic irradiation enhances the transcription of PGC-1. The PGC-1 expression-modulating device 1 according to the present invention modulates expression of PGC-1 and in turn modulates expression of VEGF. This makes it possible to regulate angiogenesis which VEGF is involved in. Therefore, the PGC-1 expression-modulating device 1 can be suitably used in the treatment of ischemic disease.

Here, by means of a conventionally known method, (a) modulation of PGC-1 expression by the PGC-1 expression-modulating device 1 according to the present invention and (b) modulation of VEGF expression, which is caused by the modulation (a), can be confirmed by measuring an expression level of PGC-1 or VEGF at a site subjected to ultrasonic irradiation. Besides, the PGC-1 expression-modulating device 1 according to the present invention may be configured such that the controller 12 gets feedback on a measured expression level so that the controller 12 controls ultrasonic wave outputs from the transmission oscillating section 142 and the transmitter 141 in accordance with the feedback.

Here, the living body that can be a subject of ultrasonic irradiation by the PGC-1 expression-modulating device 1 according to the present invention includes a living cell, a living tissue, and an animal individual(s). Assuming that the subject of ultrasonic irradiation by the PGC-1 expression-modulating device 1 according to the present invention is a living cell, a human mesenchymal stem cell (MSC) is given as an example of the living cell. Another example of the living cell as the subject of ultrasonic irradiation may include a human umbilical vein-derived endothelial cell (HUVEC), a human aorta-derived vascular smooth muscle cell (AoSMC), and a mouse myoblast cell line (C2C12).

For cases where the PGC-1 expression-modulating device 1 according to the present invention is used for animal individual(s), the probe 20 is attached to the living body so as to non-invasively contact a skin surface over the affected site via water, gel, a coupling agent, or the like medium. After the probe 20 is attached to the living body, ultrasonic waves are transmitted from the ultrasonic wave transmitting section 14 to the transducer 21, and the transducer 21 then outputs the ultrasonic waves to the living body.

Here, the ultrasonic waves to be irradiated over the living body by the PGC-1 expression-modulating device 1 preferably has a frequency in a range from 0.5 MHz to 3 MHz ± 10 %, more preferably in a range from 1 MHz to 2 MHz ± 10 %, most preferably 2 MHz ± 10 %. Note that "± 10 %" means a margin of error. That is, "in a range from 0.5 MHz to 3 MHz ± 10 %" means inclusion of (i) a range from 0.5 MHz to 3 MHz and (ii) a margin of error of plus or minus 10 % from the range (i). In other words, "0.5 MHz to 3 MHz ± 10 %" can be expressed as "0.45 MHz to 3.3 MHz". In the following descriptions regarding ranges of other numerical values, the expression "± 10 %" will be used with the same meaning.

Further, irradiation time of the ultrasonic waves is in a range from 20 minutes to 40 minutes ± 10 % per day, most preferably 20 minutes ± 10 % per day. This makes it possible to promote the PGC-1 expression more effectively. Still further, it is preferable that the ultrasonic waves to be irradiated over the living body by the PGC-1 expression-modulating device 1 has a power density of 30 mW/cm² ± 10 %, a duty ratio of 10 % ± 10 % or 20 % ± 10 %, and PRF of 1 kHz ± 10 % or 100 kHz ± 10 %. However, the present invention is not limited to this. The controller 12 controls the ultrasonic wave transmitting section 14 to irradiate ultrasonic waves over the living body under the conditions of the above-described frequency, ultrasonic irradiation time, power density, duty ratio, and PRF.

Further, the controller 12 may control the ultrasonic wave transmitting section 14 so that the transducers 21 through 21n are sequentially driven by time division to emit ultrasonic waves of the same frequency. For example, assume that ultrasonic irradiation with use of four transducers 21 is performed under the conditions where PRF is 1 kHz and a duty ratio is 20 %. In this case, one of the transducers 21 is driven for 200 microseconds and then stopped for 50 microseconds, after which another one of the transducers 21 is driven for 200 microseconds. This process is sequentially repeated with respect to all of the four transducers 21. Control is performed so that the total irradiation time and the total power density fall within the above-described ranges. The order of driving the transducers 21 is not particularly limited. The ultrasonic irradiation carried out in the above-described manner allows the living body to be efficiently irradiated with ultrasonic waves, without concentration of ultrasonic irradiation energy onto a particular spot of the living body.

### (Second Embodiment)

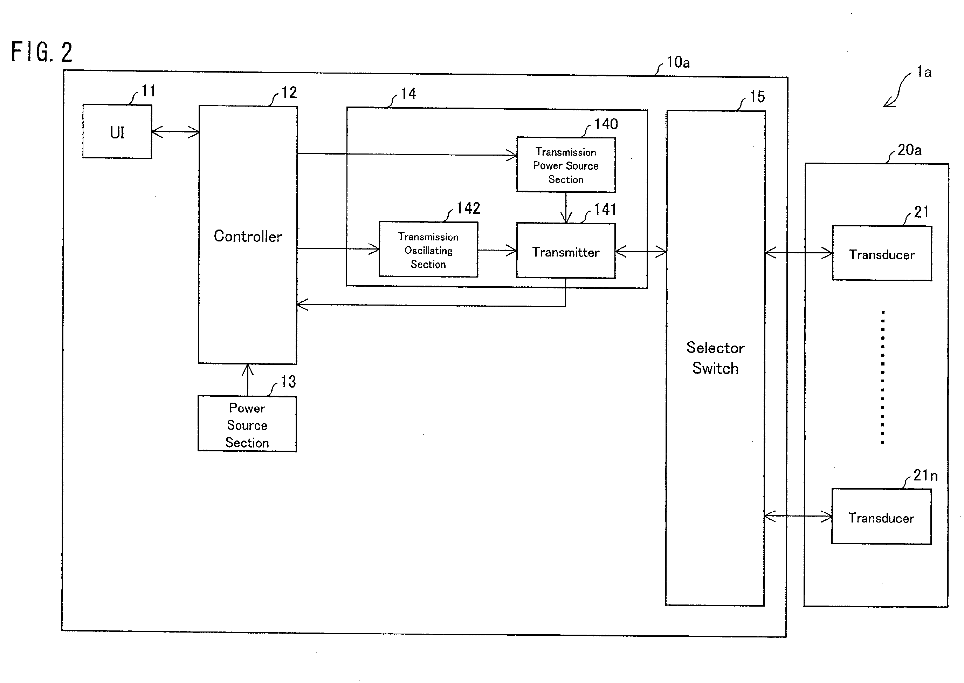

With reference to Fig. 2, the following will describe other embodiment of the PGC-1 expression modulating device according to the present invention. Fig. 2 is a block diagram showing other embodiment of a PGC-1 expression modulating device 1a according to the present invention. As shown in Fig. 2, the PGC-1 expression-modulating device 1a according to the present embodiment is different from the PGC-1 expression-modulating device 1 according to First embodiment in that the PGC-1 expression-modulating device 1a includes a single ultrasonic wave transmitting section 14 and a selector switch 15. In the descriptions of the present embodiment, only the differences from First Embodiment will be explained in detail, and explanations of the others are omitted here.

In the PGC-1 expression-modulating device 1a, the single ultrasonic wave transmitting section 14 provided in a main unit 10a transmits predetermined ultrasonic waves to a plurality of transducers 21 through 21n provided in a probe 20a. Ultrasonic waves are produced by oscillation of the transmission oscillating section 142 provided in the ultrasonic wave transmitting section 14, and then outputted to the transmitter 141. Then, the ultrasonic waves are transmitted to the transducers 21. At this time, switching is performed by the selector switch 15 so that predetermined ultrasonic waves are transmitted to the transducers 21 through 21n. Thus, by provision of the plurality of transducers 21, ultrasonic irradiation over a large area is realized, while the ultrasonic wave transmitting section 14 is reduced in number. This makes it possible to realize size reduction of the main unit 10a and low-cost ultrasonic irradiation.

### (Transducer 21)

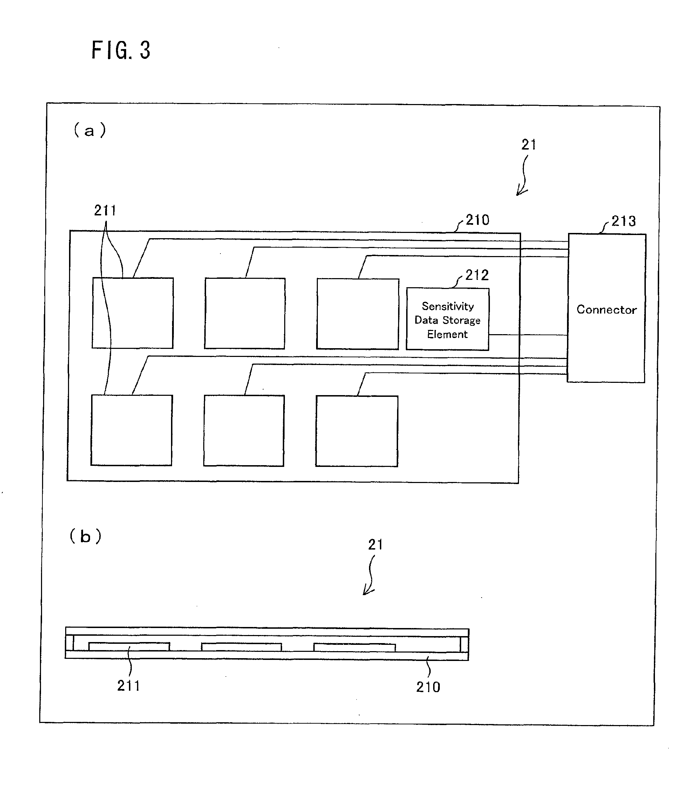

Next, with reference to (a) and (b) of Fig. 3, the following will describe the configuration of the transducer 21 included in the probe 20 or 20a of the PGC-1 expression-modulating device 1 or 1a. In Fig. 3, (a) and (b) are schematic diagrams showing examples of the configuration of the transducer 21 provided in the PGC-1 expression modulating device 1 and 1a according to the present invention. In Fig. 3, (a) is a top view of the transducer 21. In Fig. 3, (b) is a cross-sectional view of the transducer 21.

As shown in (a) and (b) of Fig. 3, the transducer 21 has a transducer housing 210 where a plurality of ultrasonic transducers 211 and a sensitivity data storage element 212 are provided. The ultrasonic transducers 211 and the sensitivity data storage element 212 are connected to each other via a connector 213. The ultrasonic transducers 211 are provided in a matrix with 2 rows and 3 columns. Note that in the present embodiment, the ultrasonic transducers 211 are provided in a matrix with 2 rows and 3 columns by using six elements. However, the present invention is not limited to this. The housing 210 is made of silicon or the like material. As shown in (a) and (b) of Fig. 3, it is preferable that the transducer 21 is in rectangular shape, so that the transducer 21 can contact the living body over a larger area, and the living body is irradiated with ultrasonic waves over a larger area. In the probe 20a, the transducer 21 in rectangular shape is provided in plural, and the adjacent transducers 21 are connected to each other.

Upon receipt of ultrasonic waves transmitted from the ultrasonic wave transmitting section 14, each of the plurality of ultrasonic transducers 211 vibrates and performs ultrasonic irradiation over the living body. The sensitivity data storage element 212 stores and holds sensitivity data on the transducer 21. The sensitivity data held for each of the transducers 21 is given as a feedback to the controller 12 or the transmitter 141. In accordance with the feedback, variations in sensitivity of the transducers are corrected. Further, the transducer 21 may include an element which stores data that indicates a sensitivity level of the transducer. Such data may be given as a feedback to the controller 12 or the like so that variations in sensitivity are corrected.

### [System for treating ischemic disease]

A system for treating ischemic disease includes the above-described PGC-1 expression-modulating device 1 according to the present invention.

A disease to be treated by the system for treating ischemic disease according to the present invention is ischemic disease caused by decreased blood flow. However, the disease to be treated by the device and method for treating ischemic disease is not particularly limited as long as the disease can be effectively treated by modulation of PGC-1 expression. Examples of such disease include angina pectoris, myocardial infarction, cerebral infarction, arteriosclerosis obliterans, chronic arteriosclerosis obliterans (ASO), and Burger's disease (TAO). In particular, the system for treating ischemic disease according to the present invention can be suitably used in treatments of severe ischemic peripheral vascular diseases such as ASO and TAO.

In the system for treating ischemic disease according to the present invention, a site of ischemic disease (affected site) of a living body, which is a subject of the treatment, is irradiated with ultrasonic waves so that PGC-1 expression is modulated, thus promoting angiogenesis in which VEGF is involved. In this manner, the above-described ischemic diseases are treated. The modulation of PGC-1 expression by the system for treating ischemic disease according to the present invention can be realized by causing an affected site of the living body to be irradiated with ultrasonic waves from the ultrasonic wave transmitting section 14 of the PGC-1 expression-modulating device 1 according to the present invention.

In the system for treating ischemic disease according to the present invention, the ultrasonic waves to be irradiated over the living body has a frequency in a range from 0.5 MHz to 3 MHz ± 10 %, more preferably in a range from 1 MHz to 2 MHz ± 10 %, most preferably 2 MHz ± 10 %. Further, irradiation time of the ultrasonic waves is in a range from 20 minutes to 40 minutes ± 10 % per day, most preferably 20 minutes ± 10 % per day. Still further, it is preferable that the ultrasonic waves to be irradiated over the living body have a power density of 30 mW/cm² ± 10 %, a duty ratio of 10 % ± 10 % or 20 % ± 10 %, and PRF of 1 kHz ± 10 % or 100 kHz ± 10 %. However, the present invention is not limited to this.

Yet further, ultrasonic waves sequentially emitted from a plurality of transducers by time division may be irradiated over the living body. This makes it possible to efficiently irradiate the living body with ultrasonic waves, without concentration of ultrasonic irradiation energy onto a particular spot of the living body.

The system for treating ischemic disease according to the present invention can be used with the probe 20 of the PGC-1 expression-modulating device 1 attached to the living body in contact with a skin surface over the affected site. After the probe 20 is attached to the living body, ultrasonic waves are transmitted from the ultrasonic wave transmitting section 14 to the transducer 21, and the transducer 21 then outputs the ultrasonic waves to the living body.

In the method for treating ischemic disease, it is preferable that the ultrasonic waves are irradiated over a whole affected area of the living body. In the case where the affected site to be treated is too large to be irradiated with ultrasonic waves at a single irradiation, irradiations are carried out in the following manner. In this case, the probe 20 is detached after first ultrasonic irradiation, and the probe is then attached to other spot for second ultrasonic irradiation. This action is repeated until the whole affected area is irradiated with the ultrasonic waves. Note that the wording "affected site" herein means a site where the disease occurs (site where a lesion is observed), and the wording "whole affected area" herein means a certain area that contains all of the sites where the disease occurs.

The method for treating ischemic disease may further include a step of confirming a treatment effect. In such a step, the treatment effect may be confirmed by measuring an expression level of PGC-1 or VEGF in a site subjected to ultrasonic irradiation.

According to the method for treating ischemic disease, ultrasonic waves are non-invasively irradiated over the affected site to modulate PGC-1 expression, which promotes angiogenesis in which VEGF is involved. This enables treatment without excessive burden on a patient. Besides, a patient who could use the treatment is not limited because the cost of the treatment is low, safety of the treatment is established, and the treatment involves a high level of safety.

### [Examples]

### [Example 1]

Using human mesenchymal stem cells (MSC), changes in the expression levels of PGC-1 and VEGF by ultrasonic irradiation were investigated.

Human mesenchymal stem cells (R17, R22, R37, R44) (collected from iliac bone marrow of a patient) were cultured for 48 hours on a 6-well plate so as to be subconfluent. After that, the cells were maintained in serum-starvation (0.1% BSA) for 15 hours.

Next, the cells were inoculated on two 10cm-dishese and subcultured with a low-glucose DMEM medium (manufactured by SIGMA; D6046) containing 10% FBS (manufactured by Biowest). Thereafter, the subcultured cells were divided into five dishes. When the cells reached 95% confluence, the medium was removed, and the cells were washed with PBS. The cells washed were then mixed with 2 mL of 0.05% trypsin/0.02% EDTA, and allowed to leave for 3 minutes. The mixture was mixed with 8 mL of low-glucose DMEM medium (manufactured by SIGMA; D6046) containing 10% FBS (manufactured by Biowest), and 50 mL of cell suspension was transferred to a tube.

The tube containing the cell suspension was centrifuged (for 5 minutes at 1100 rpm), after which a supernatant thereof was removed. The cells were resuspended in an appropriate amount of low-glucose DMEM medium (manufactured by SIGMA; D6046) containing 10% FBS (manufactured by Biowest) so as to be 50 % confluent. The resultant suspension was inoculated on a 6-well plate by 2 mL per well and cultured in a CO₂ incubator. Two days after the culturing, the medium was removed, and the cells were washed twice with PBS. After that, the cells were mixed with 2 mL of low-glucose DMEM medium containing 0.1% BSA and maintained in serum-starvation.

Sixteen hours after the maintaining in serum-starvation, ultrasonic irradiation using the device, shown in Fig. 1, with four transducers 21 was carried out for 20 minutes with respect to the cells of ultrasonic irradiation groups under the following conditions: frequencies (1MHz, 1.5MHz, 2MHz and 3MHz), a power density (30 mW/cm²), a duty ratio (20 %), and PRF (1kHz). The ultrasonic irradiation was carried out in the following manner. That is, the first transducer 21 was driven for 200 microseconds and stopped for 50 microseconds. After that, the second transducer 21 was driven for 200 microseconds. This process was sequentially repeated using all the four transducers 21 so that the total irradiation time and total power densities became the above-described values. More specifically, the device shown in Fig. 1 was placed on a vat, and the vat was filled with water. The 6-well plate on which the cell suspension was inoculated was floated on water in the vat. In this manner, the device was coupled to water. Then, the cells were irradiated with ultrasonic waves by the device. One hour, 3 hours, 6 hours, 9 hours, and 12 hours after the ultrasonic irradiation was started, the medium was removed, and the cells were washed twice with PBS.

After washing, the cells were mixed by pipette with 0.6 mL of Solution I (Roche RNA Isolation Kit, manufactured by Roche). After that, the cells were collected, and resultant samples were frozen at -80 °C until RNA extraction was carried out. In the same fashion, preparation was made for control cells to which no ultrasonic waves were applied.

From the frozen samples, total RNA was extracted, using High Pure RNA Isolation Kit, according to Roche RNA Isolation Kit manual.

On a reaction plate, 500 µg of RNA thus extracted was placed, and RNase/DNase-free water was added thereto so that a liquid volume became 10µL. Further, 1 µL of OLIGO dT20 (2 pmol/µL) was further added thereto, and RNA was denatured for 5 minutes at 65 °C. After the RNA denaturation, the plate was rapidly cooled to room temperature. After that, the plate was centrifuged for 1 minute. Further, 9 µL of RT-PCR reaction solution shown in Table 1 was added thereto, and the mixture solution was stirred and centrifuged. RT-PCR was carried out at 42 °C for 20 minutes and at 99 °C for 5 minutes. For reverse transcriptase, ReverTraAce® (manufactured by TOYOBO) was used.

**(Table 1)**

| | Addition amount per sample (µL) | The number of samples | Total (µL) |
|---|---|---|---|
| Nuclease-free distilled water | 1.75 | 50 | 87.5 |
| 5 x RT buffer | 4 | 50 | 200 |
| dNTP mixture solution (each 10 mM) | 2 | 50 | 100 |
| RNase inhibitor (40 U/µL) | 0.25 | 50 | 12.5 |
| ReverTra Ace® | 1 | 50 | 50 |
| Total | 9 | 50 | 450 |

The reaction solution thus obtained after the RT-PCR step was centrifuged, 80 µL of RNase/DNase-free water was added thereto, and the mixture solution was stirred. Then, 2 µL of cDNA thus obtained was placed in each well of a plate to which 8 µL of real-time PCR reaction solution shown in Table 2 was added, and the mixture solution was stirred. After that, the plate was centrifuged at 4000 rpm for 10 minutes. Then, real-time PCR was carried out in the following manner. The reaction solution thus obtained by centrifugation was allowed to react for 10 minutes at 95 °C. After that, the reaction solution was allowed to react for 10 seconds at 95 °C and for 25 seconds at 60 °C up to a maximum of 45 cycles. Real-time PCR was carried out by using LightCycler 48011. From cDNAs amplified by real-time PCR, RNA expression levels of PGC-1α, VEGF-A, VEGF-B, and VEGF-C were quantified with respect to an internal standard gene, HPRT1.

**(Table 2)**

| | Addition amount per sample (µL) | The number of samples | Total (µL) |
|---|---|---|---|
| Primer (left) | 0.1 | 50 | 5 |
| Primer (right) | 0.1 | 50 | 5 |
| UniversalProbe Library probe | 0.1 | 50 | 5 |
| DNase/ RNase-free water | 2.7 | 50 | 135 |
| ROX Mix | 5.0 | 50 | 250 |
| Total | 8.0 | 50 | 400 |

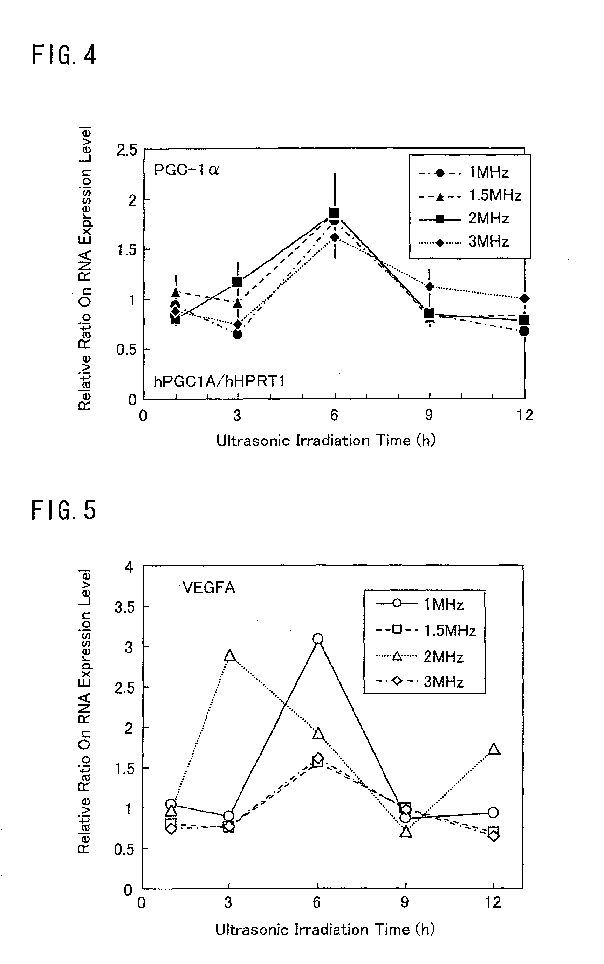

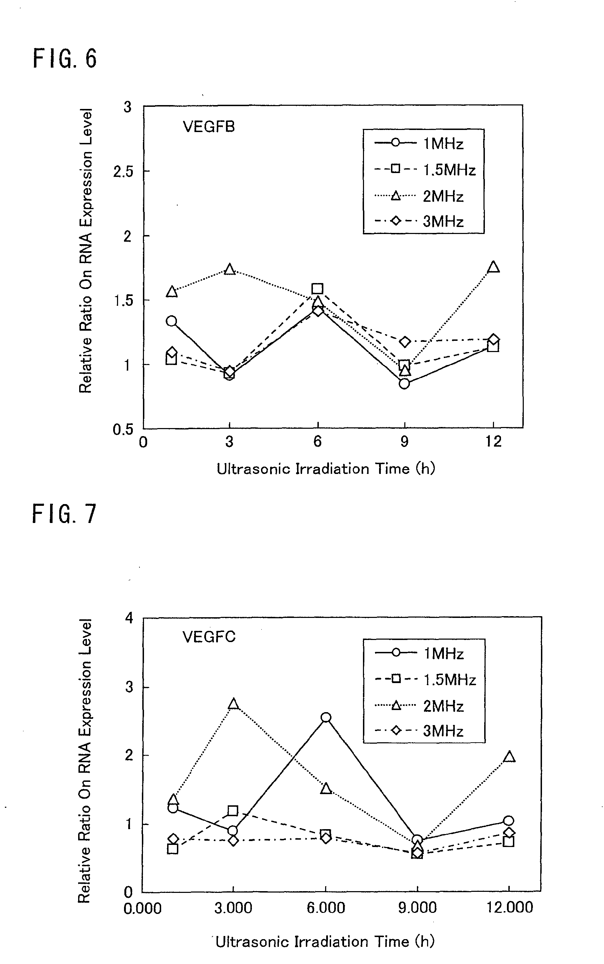

The results are shown in Figs. 4 through 7. In Figs. 4 through 7, relative ratios on gene transcription levels of the cells after the ultrasonic irradiation are shown, with respect to gene transcription levels in the control cells that were not subjected to ultrasonic irradiation.

As shown in Fig. 4, a relative ratio on RNA expression level of PGC-1α in the cells to which ultrasonic waves were applied, when RNA expression level of PGC-1α in the control cells (compared to HRPT1) was 1, increased at ultrasonic frequencies of 1 MHz to 3 MHz until 6 hours after the ultrasonic irradiation. As shown in Figs. 5 through 7, RNA expression levels of VEGF-A, VEGF-B, and VEGF-C also increased by ultrasonic irradiation.

### (Comparative Example 1)

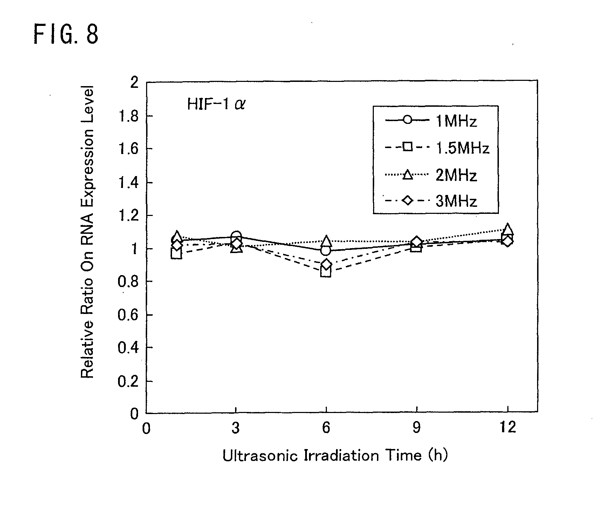

In human mesenchymal stem cells prepared in the same manner as in Example 1, change in HIF-1α expression level by ultrasonic irradiation was investigated in the same manner as in Example 1. The result is shown in Fig. 8. As shown in Fig. 8, RNA expression level of HIF-1α was not changed by ultrasonic irradiation.

### (Example 2)

Using angiogenesis model mice, changes in PGC-1 and VEGF expression levels by ultrasonic irradiation were investigated.

Rabbit anti-PGC-1 antibodies (Sc-13067), rabbit anti-VEGF antibodies (sc-152), goat anti-VEGF-B antibodies (sc-1876), and goat anti-VEGF-C antibodies (sc-1881) were purchased from Santa Cruz Biotechnology, Inc. Anti-CD31 antibodies were purchased from BD Pharmingen. Histofine® simple stain mouse MAX-PO (R), Histofine® simple stain mouse MAX-PO (Goat), simple stain DAB solution were purchased from purchased from Nichirei Bioscience Inc. An alkaline phosphatase staining kit was purchased from Sigma-Aldrich.

A mouse (C57B6/6J, male, 8 weeks old) was purchased from Charles River Japan Co., Ltd. The preparation of the angiogenesis model mouse was carried out according to a standard method known in this art (Reference Literature 1: Am J Pathol 1998 152:1667-1679). A leg (affected leg) of the angiogenesis model mouse was subjected to ischemic treatment, and a coupling agent was applied to the leg. Ultrasonic waves were applied to the affected leg by the device shown in Fig. 1 via the coupling agent. Ultrasonic irradiation was carried out under the same irradiation conditions as in Example 1, except that frequencies of the ultrasonic waves for irradiation were 1 MHz and 2 MHz. On the day before the ultrasonic irradiation, and on the 1st day, the 4th day, the 7th day, the 11th day, the 14th day, the 18th day, the 21st day, the 25th day, and the 28th day after the day on which the ultrasonic irradiation was started, the restoration of blood flow was assessed by laser Doppler blood flow analysis (Moor LDI; Moor Instruments, Axminster, UK).

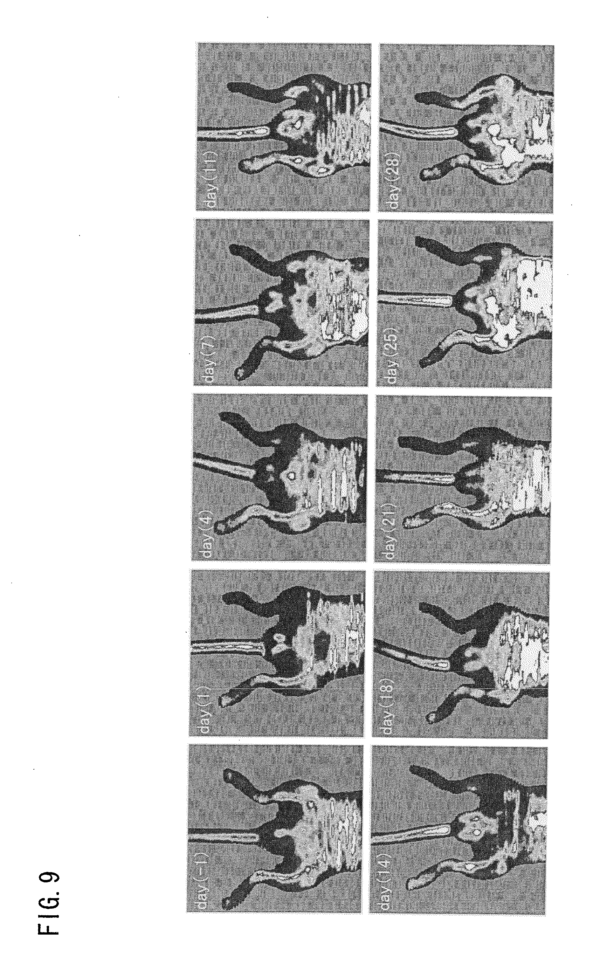

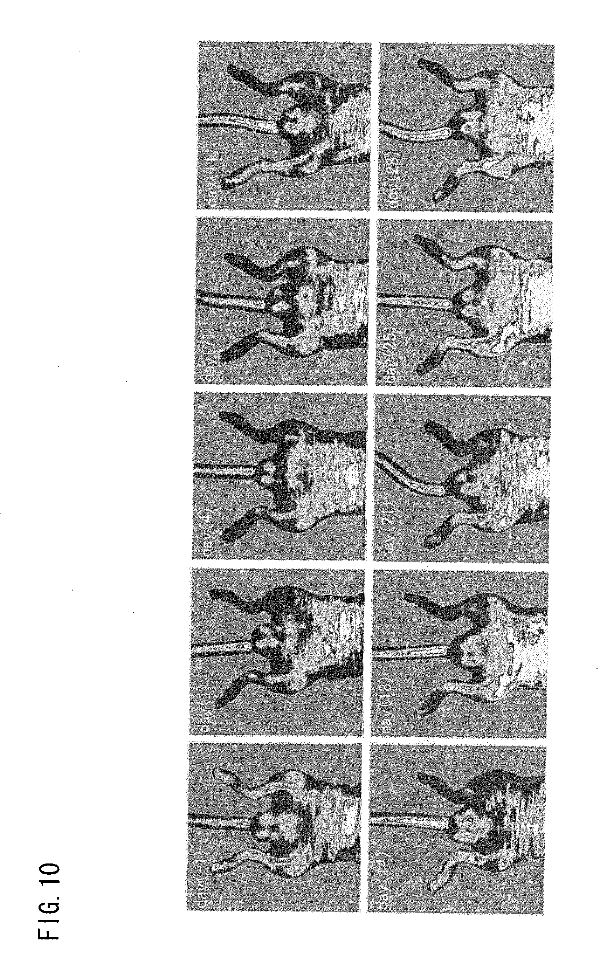

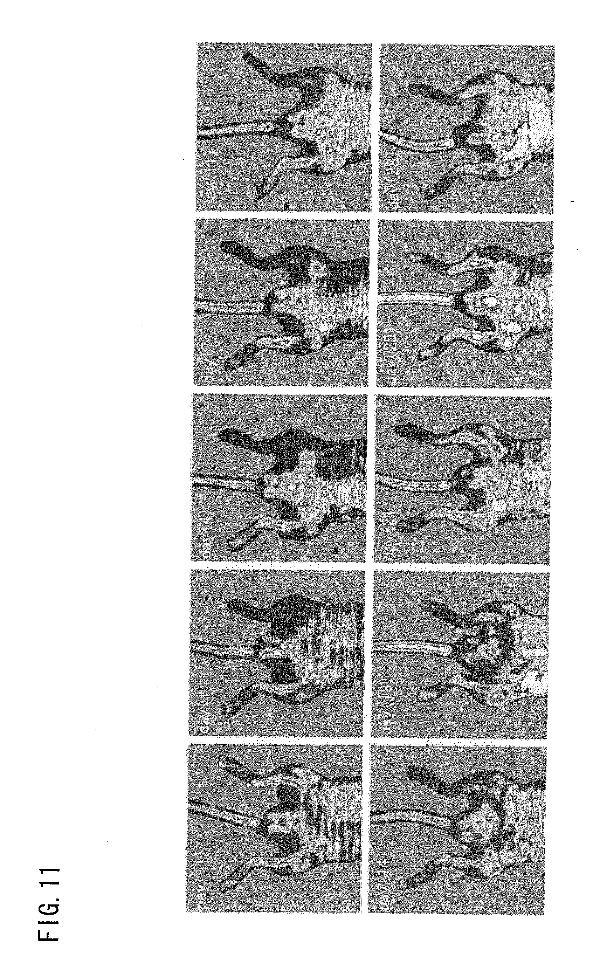

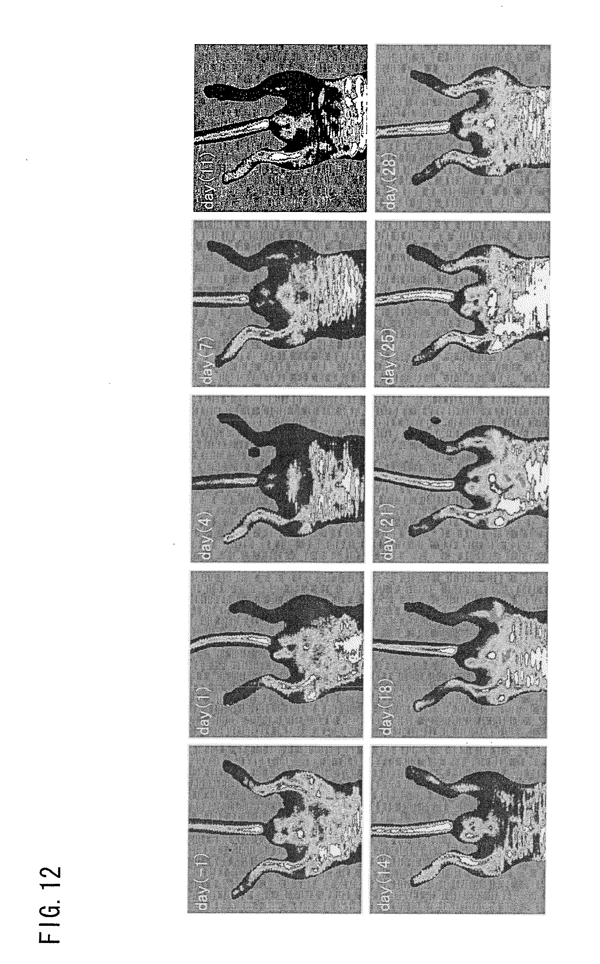

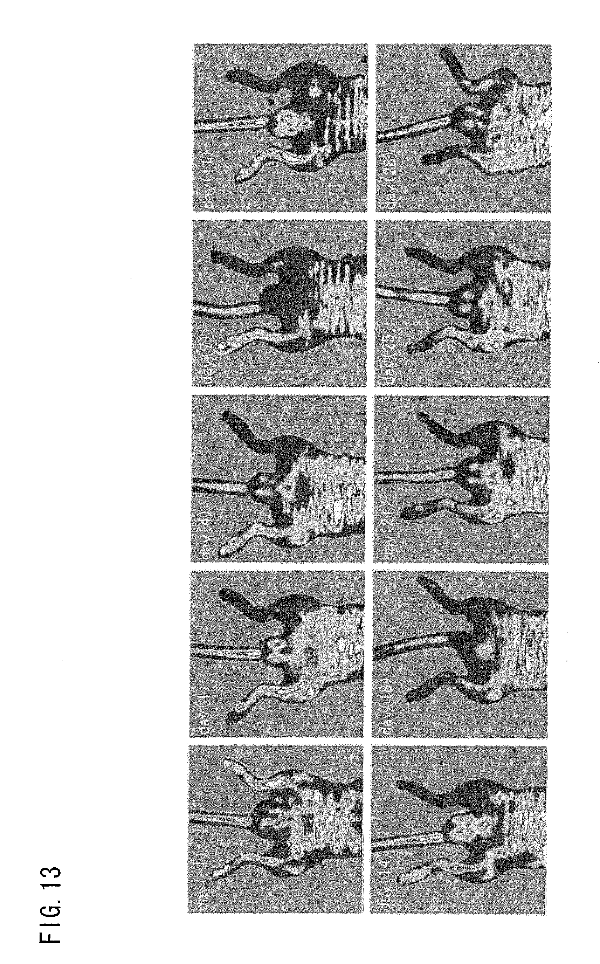

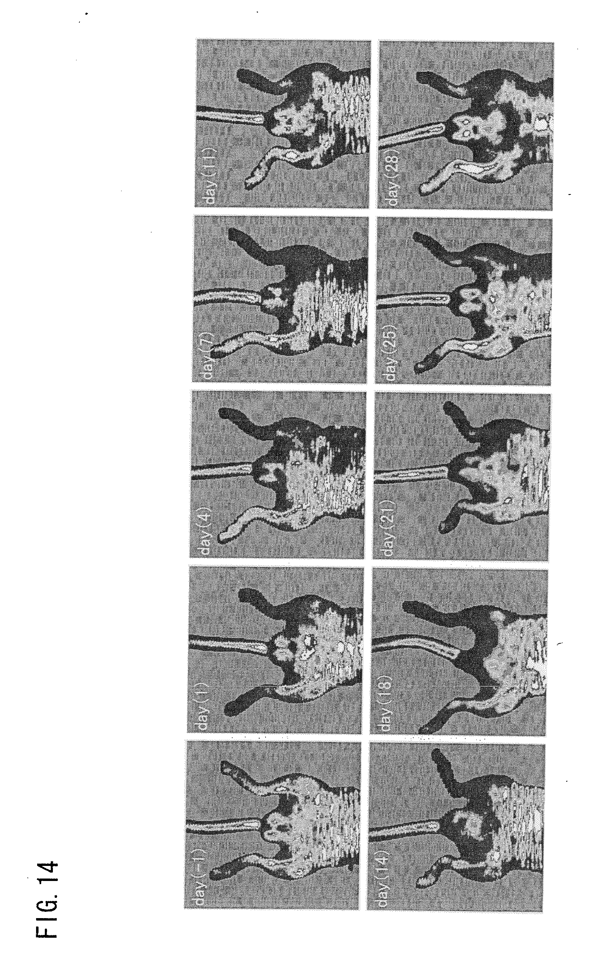

The results are shown in Figs. 9 through 14. Fig. 9 shows the result for an individual ID 10 which was irradiated with ultrasonic waves at a frequency of 1 MHz. Fig. 10 shows the result for an individual ID15 which was irradiated with ultrasonic waves at a frequency of 1 MHz. Fig. 11 shows the result for an individual ID20 which was irradiated with ultrasonic waves at a frequency of 2 MHz. Fig. 12 shows the result for an individual ID21 which was irradiated with ultrasonic waves at a frequency of 2 MHz. Fig. 13 shows the result for a control individual ID26 which was not irradiated with ultrasonic waves. Fig. 14 shows the result for a control individual ID7 which was not irradiated with ultrasonic waves.

As shown in laser Doppler image photographs of Figs. 9 through 12 for the individuals ID10 and ID15 that were irradiated with ultrasonic waves at a frequency of 1 MHz and the individuals ID20 and ID21 that were irradiated with ultrasonic waves at a frequency of 2 MHz, adequate restoration of blood flow was confirmed within 28 days after the starting day of the ultrasonic irradiation. On the other hand, as shown in laser Doppler image photographs of Figs. 13 and 14 for the control individuals ID26 and ID7 that were not irradiated with ultrasonic waves, adequate restoration of blood flow was not confirmed within 28 days after the starting day of the ultrasonic irradiation.

Using a gas anesthetic system (produced by DS Pharma) for laboratory animals, the mouse was treated under inhalation anesthesia with isoflurane (manufactured by Abbott Japan Co., Ltd.). On the 28th day, after the laser Doppler blood flow analysis was carried out, the mouse was caused to euthanasia by cutting a carotid artery of the mouse and removing blood from the mouse under anesthesia with pentobarbital (25 mg/kg). After that, thigh muscles of both legs were removed promptly and placed on a 5 mm-cork (ϕ21 mm) with 6 % tragacanth gum mounted thereon. Then, the thigh muscles were quickly frozen in isopentane cooled with liquid nitrogen. A frozen tissue thus obtained was preserved in a plastic bottle and stored at -80 °C until sliced.

### <Hematoxylin-eosin staining>

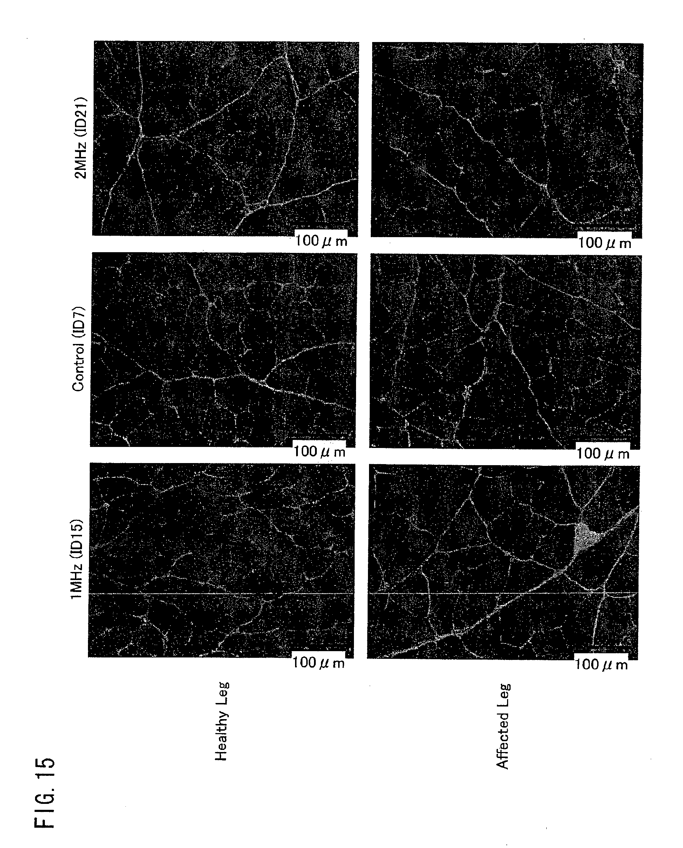

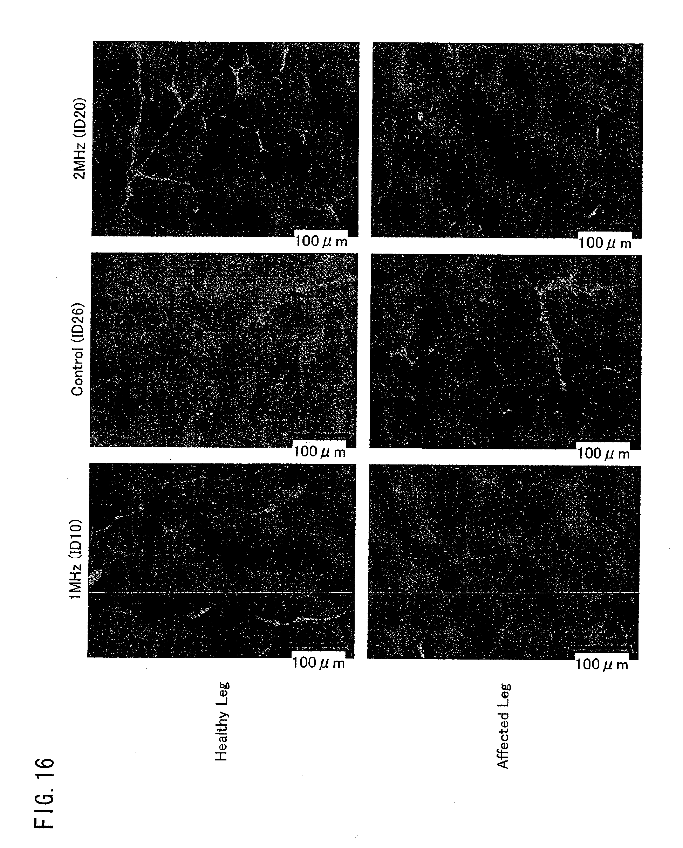

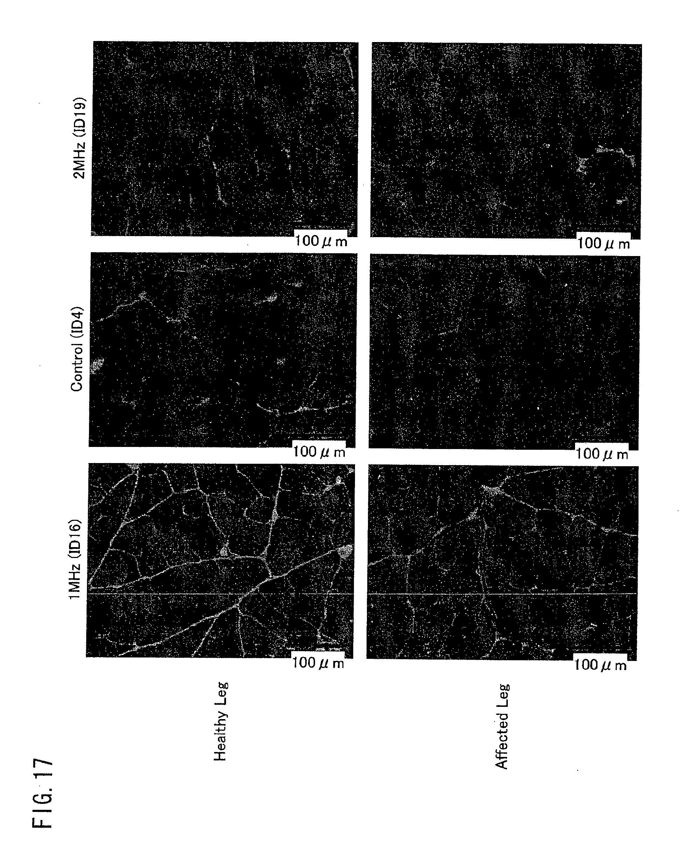

The frozen tissue being stored at -80 °C was returned to -25 °C in cryostat (LEICA CM1950) and sliced into thin sections of 5 µm in thickness. A thin tissue section thus obtained was adhered on an APS coated glass slide (S8441; manufactured by Matsunami Glass Industry Co., Ltd.). The tissue section was dried in air at room temperature for 30 minutes and then fixed in 10% neutral buffered formalin solution (062-01661; manufactured by Wako Pure Chemical Industries, Ltd.) for 10 minutes. After stained with Mayer's hematoxylin (manufactured by Sakura Finetek Japan Co., Ltd.) for 12 minutes, the fixed tissue section was colored with warm water. Further, the fixed tissue section was stained with eosin (Sakura Finetek Japan Co., Ltd.) for 10 minutes. After that, the tissue section thus obtained was washed in running water. The tissue section thus stained was subjected to dehydration and clearing treatment and then sealed. The tissue section thus sealed was micrographed by an inverted optical microscope (OLYMPUS IX81). The results are shown in Figs. 15 through 17.

As shown in Figs. 15 through 17, a muscle fiber cross-section was displayed in each sample, and vascular tissue was also confirmed. Therefore, using the samples of these individuals, alkaline phosphatase staining and immunohistochemical staining were carried out as below, and angiogenesis was observed. In addition, in order to confirm increase in the number of blood vessels, increase of CD31, which is a vascular endothelial marker, was observed by immunohistochemical staining.

### <Alkaline phosphatase staining>

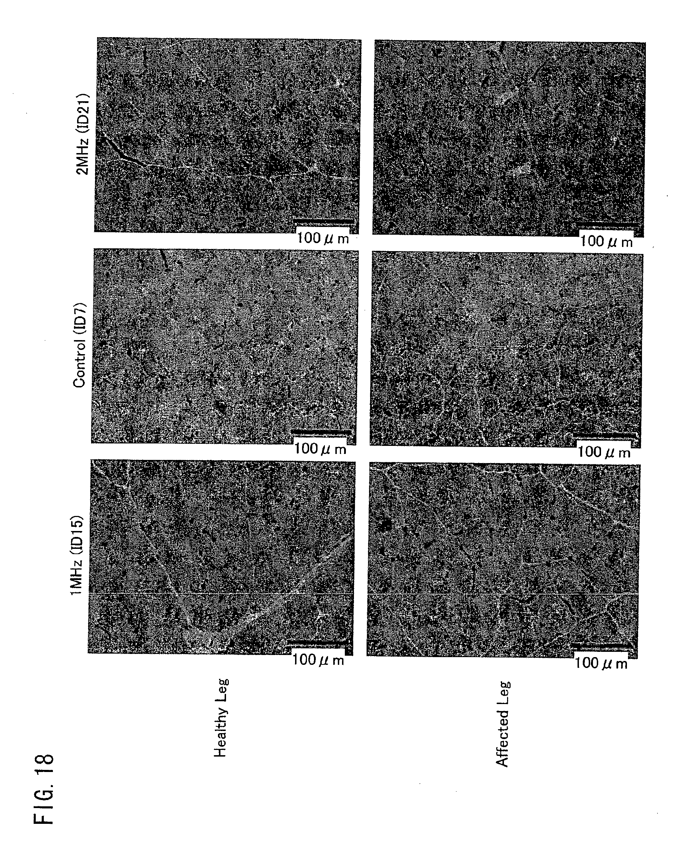

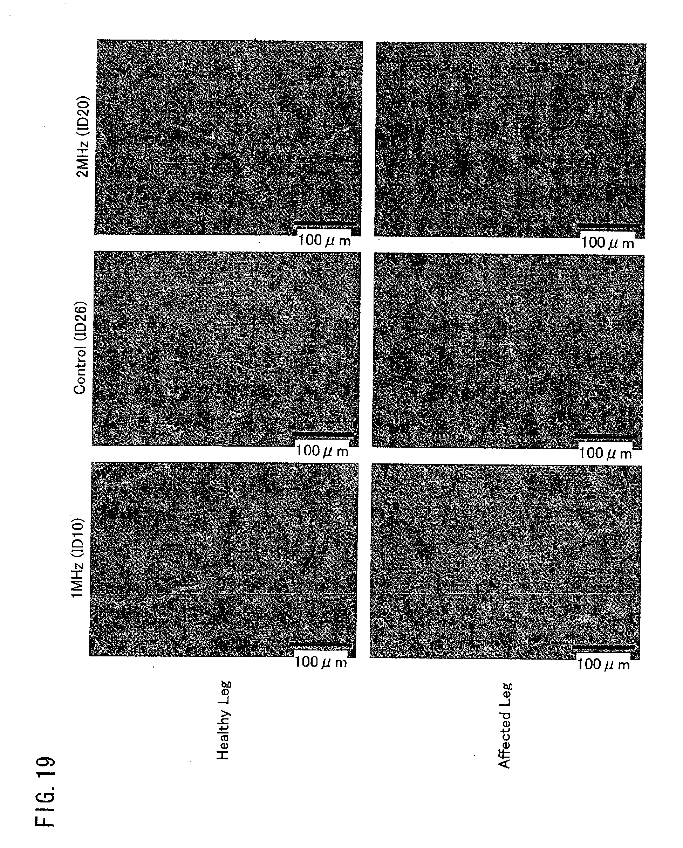

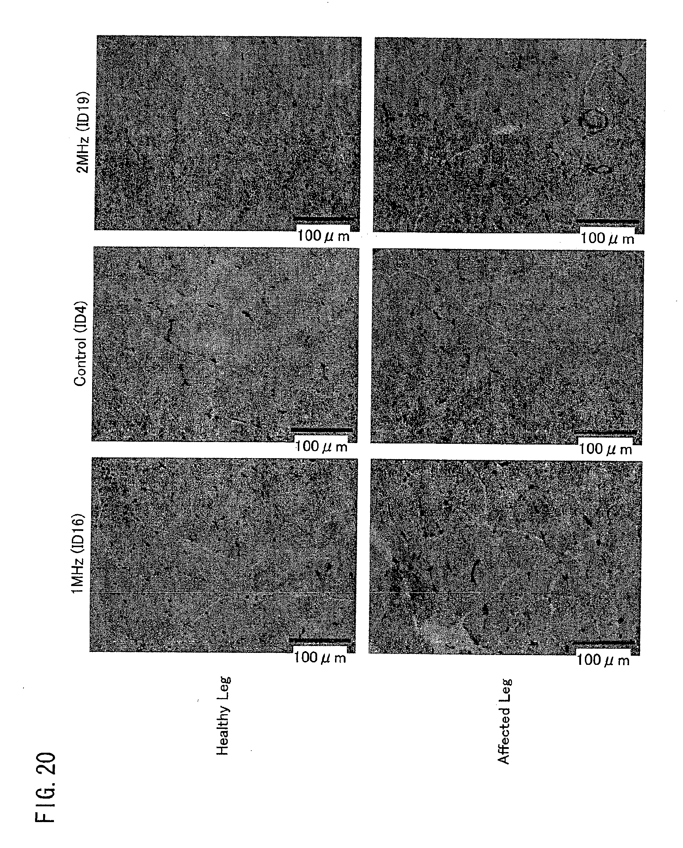

The frozen tissue being stored at -80 °C was returned to -25 °C in cryostat (LEICA CM1950) and sliced into thin sections of 5 µm in thickness. A thin tissue section thus obtained was adhered on an APS coated glass slide (S8441; manufactured by Matsunami Glass Industry Co., Ltd.). The tissue section was dried in air at room temperature for 30 minutes and was subjected to alkaline phosphatase staining by using an alkaline phosphatase staining kit according to a standard method known in this art. The tissue section thus stained was micrographed by an inverted optical microscope (OLYMPUS IX81) (Figs. 18 through 20).

As shown in Figs. 18 through 20 for the individuals ID15, ID10, and ID16 that were irradiated with ultrasonic waves at a frequency of 1 MHz and the individuals ID21, ID20, and ID19 that were irradiated with ultrasonic waves at a frequency of 2 MHz, angiogenesis was observed. On the other hand, as for the control individuals ID7, ID26, and ID4 that were not irradiated with ultrasonic waves, angiogenesis was not observed.

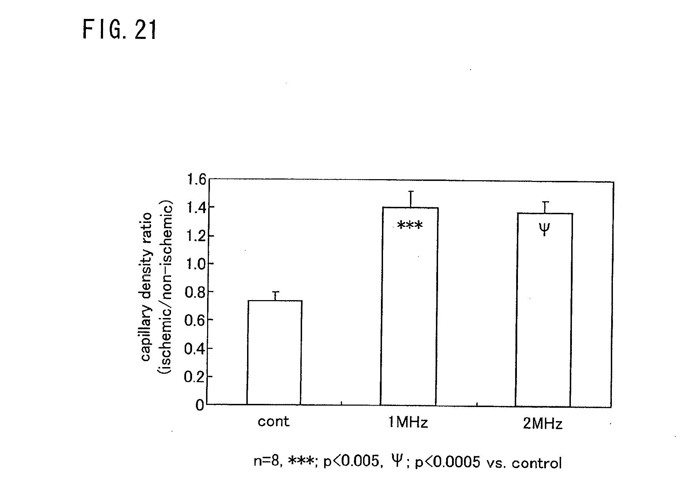

In addition, counting of spots that reacted positively to ALPase in the obtained photographic image was carried out. The number of spots thus obtained was divided by the number of muscle fibers included in the same photographic image. The division was carried out for 5 views per leg, and an average value was calculated from the values obtained for the 5 views per leg. Then, the average value obtained for the affected leg was divided by the average value for the other leg (healthy leg) that was not subjected to ischemic treatment. This is how a normalization process was carried out. The results are shown in Figure 21.

Fig. 21 is a graph of a capillary density ratio of the affected leg to a healthy leg. As shown in Fig. 21, a large amount of angiogenesis was confirmed in the ultrasonic irradiation group. On the other hand, a small amount of angiogenesis was confirmed in the control individuals, ID7, ID26, and ID4, which were not irradiated with ultrasonic waves.

### <Immunohistochemical staining>

The frozen tissue (ischemic extremity) being stored at -80 °C was returned to -25 °C in cryostat (LEICA CM1950) and sliced into thin sections of 5 µm in thickness. A thin tissue section thus obtained was adhered on an APS coated glass slide (S8441; manufactured by Matsunami Glass Industry Co., Ltd.). The tissue section was dried in air at room temperature for 30 minutes and then fixed in 10% neutral buffered formalin solution for 10 minutes. The tissue section was thoroughly washed with TBST and subjected to 1.5 % hydrogen peroxide water-added methanol treatment to inactivate intrinsic peroxidase. After thoroughly washed with TBST, blocking treatment was carried out for 1 hour at room temperature with use of 0.5 % blocking reagent (1096176: Roche Diagnostics Corporation) dissolved in TBST.

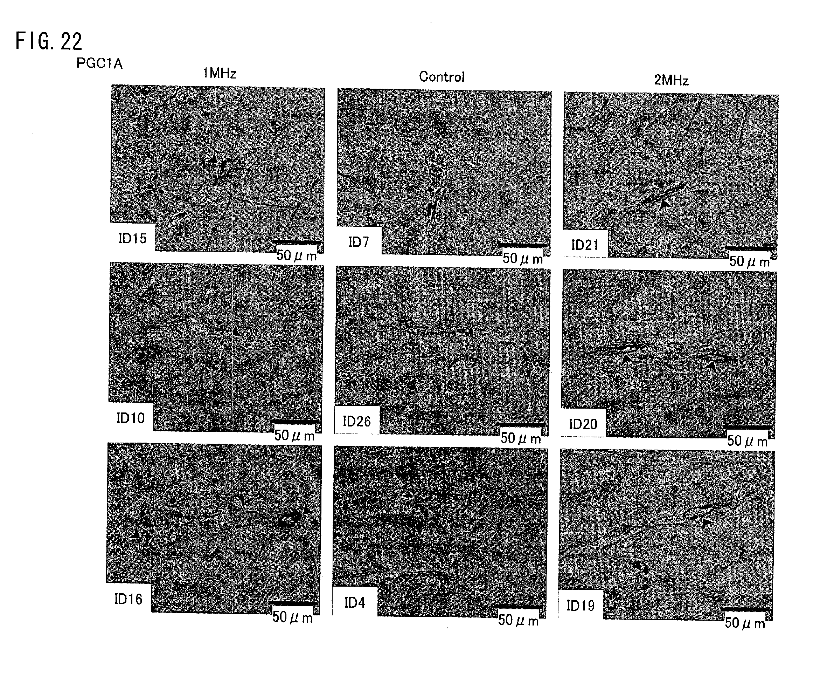

Various kinds of primary antibodies appropriately diluted with 0.5 % blocking reagent/TBST were dropped on the tissue section, and antigen-antibody reaction was carried out overnight at 4 °C. After thoroughly washed with TBST, the tissue section was treated by using various kinds of histofine simple stains according to a standard method known in this art. On the tissue section thoroughly washed with TBST, a simple stain DAB solution was dropped, and the tissue section was allowed to react at room temperature for 5 to 20 minutes. After that, the tissue section was washed in running water to stop the reaction. After more thoroughly washed with purified water, the tissue section was allowed to react with 5-fold diluted Mayer's hematoxylin for 30 seconds and was colored with warm water. The tissue section thus stained was subjected to dehydration and clearing treatment and then sealed. The tissue section thus sealed was micrographed by an inverted optical microscope (OLYMPUS IX81). The results are shown in Figs. 22 through 27.

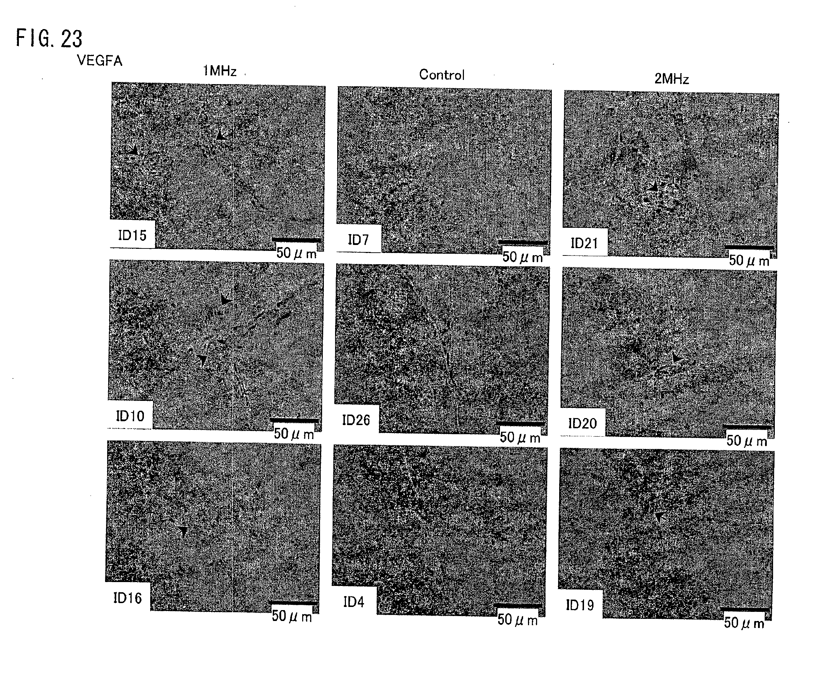

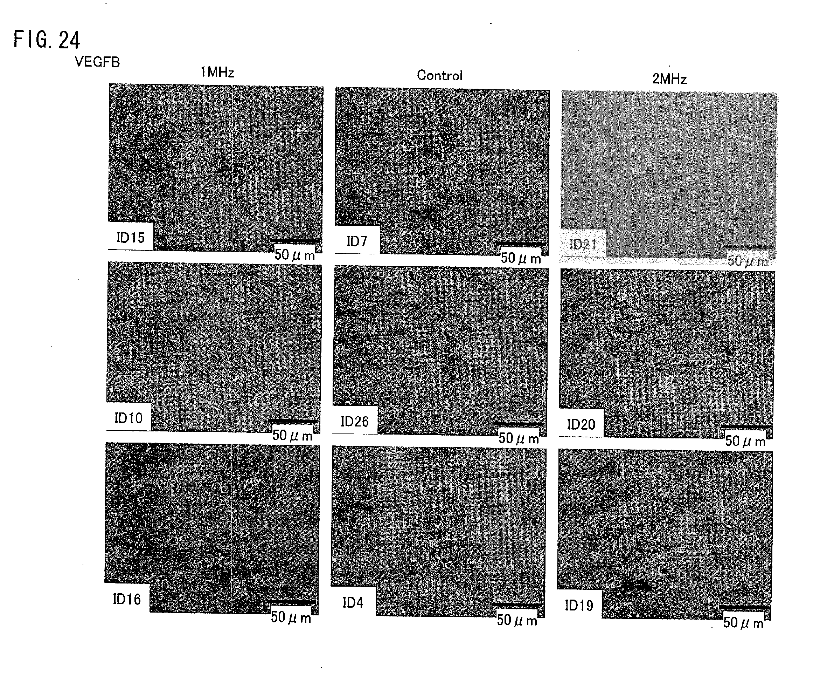

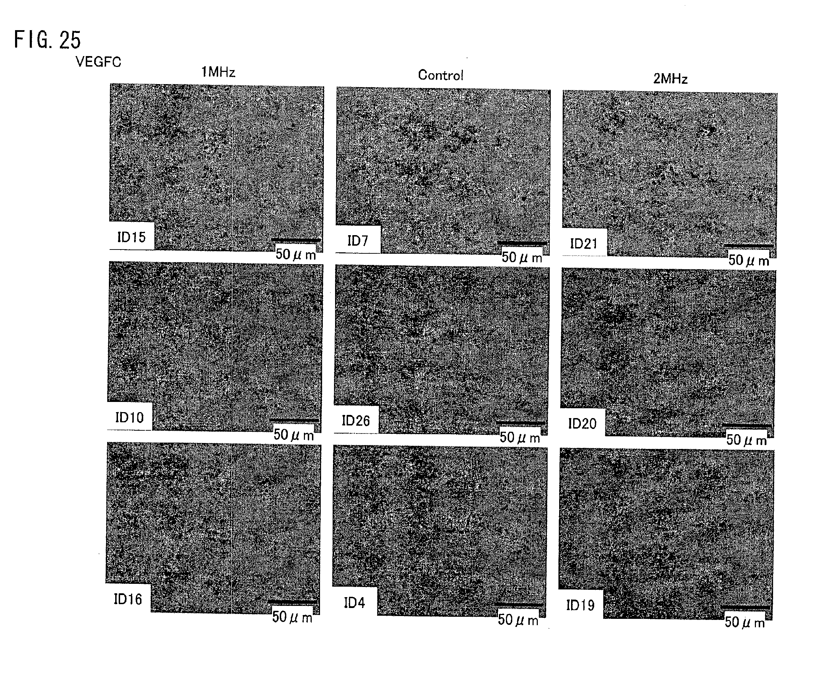

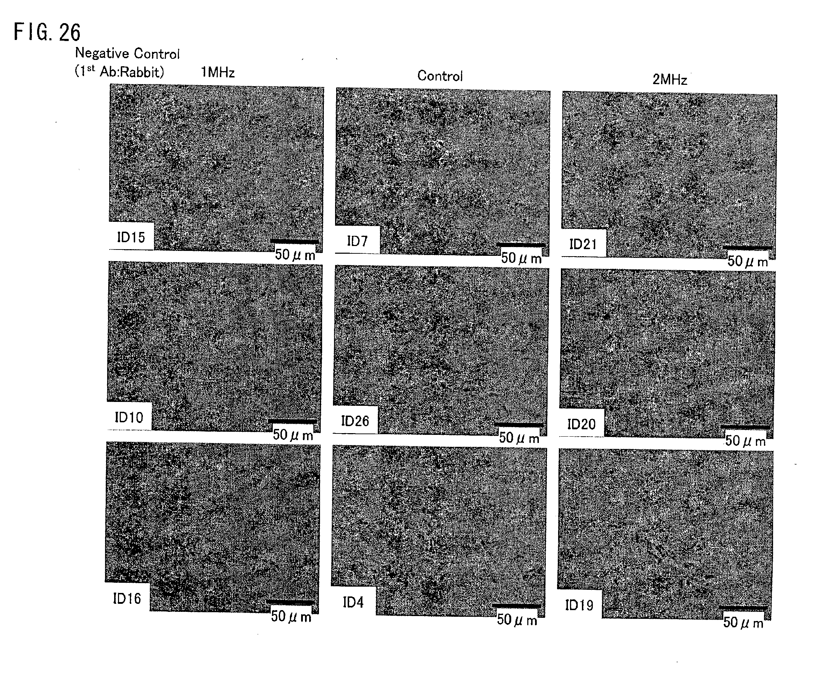

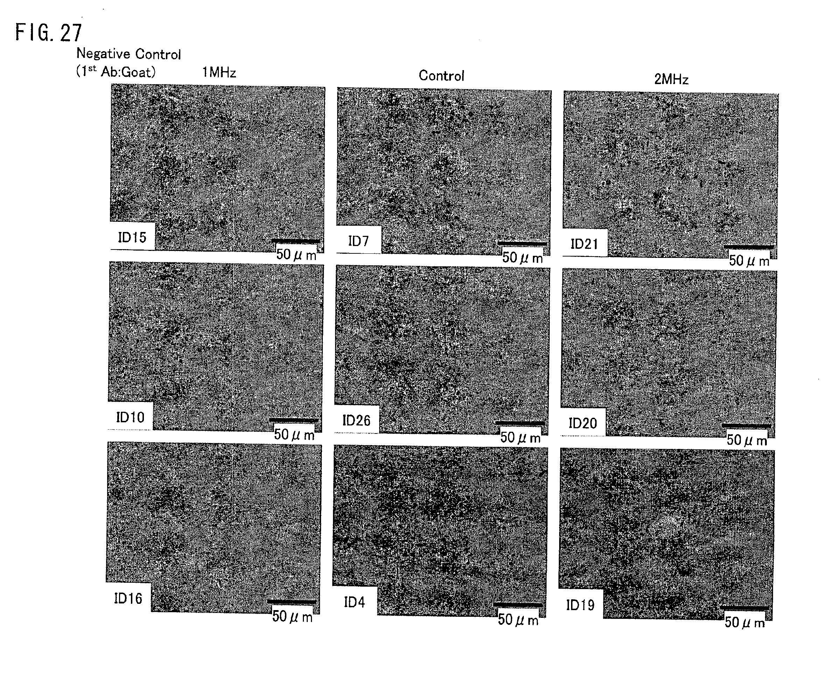

Fig. 22 is a photograph drawing showing the result of confirmation of PGC-1α expression using a rabbit anti-PGC-1 antibody. Fig. 23 is a photograph drawing showing the result of confirmation of VEGF-A expression using a rabbit anti-VEGF-A antibody. Fig. 26 shows the result of using a rabbit negative control antibody as a negative control against these antibodies. Fig. 24 is a photograph drawing showing the result of confirmation of VEGF-B expression using a goat anti-VEGF-B antibody. Fig. 25 is a photograph drawing showing the result of confirmation of VEGF-C expression using a goat anti-VEGF-C antibody. Fig. 27 shows the result when a goat negative control antibody was used as a negative control against these antibodies.

As shown in Figs. 22 through 25, PGC-1α expression, VEGF-A expression, VEGF-B expression, and VEGF-C expression were confirmed in the individuals ID15, ID10, and ID16 that were irradiated with ultrasonic waves at a frequency of 1 MHz and the individuals ID21, ID20, and ID19 that were irradiated with ultrasonic waves at a frequency of 2 MHz. On the other hand, PGC-1α expression and VEGF-A expression in the control individuals ID7, ID26, and ID4 that were not irradiated with ultrasonic waves were less than those in the individuals that were irradiated with ultrasonic waves. VEGF-B expression and VEGF-C expression in the control individuals ID7, ID26, and ID4 were equal to those in the individuals that were irradiated with ultrasonic waves.

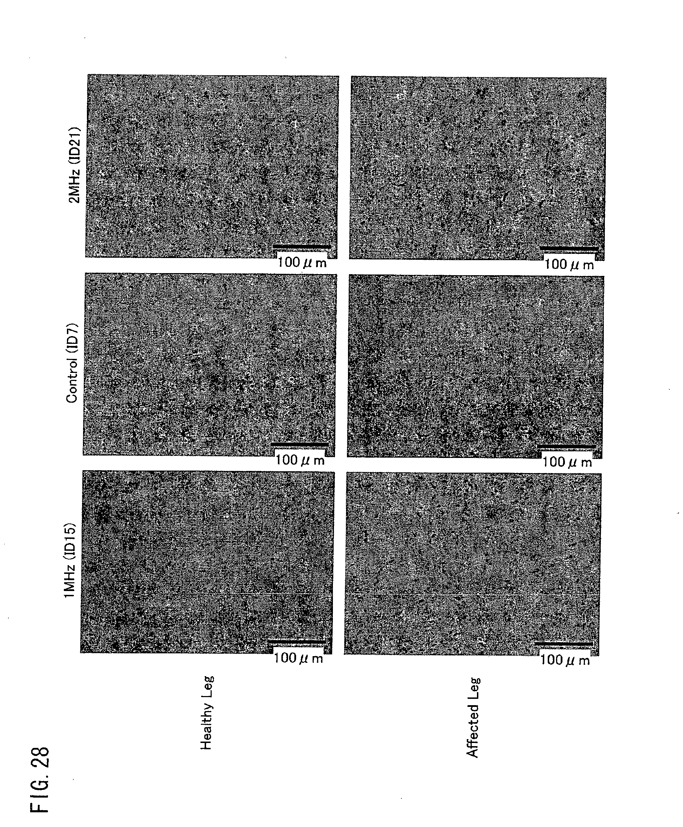

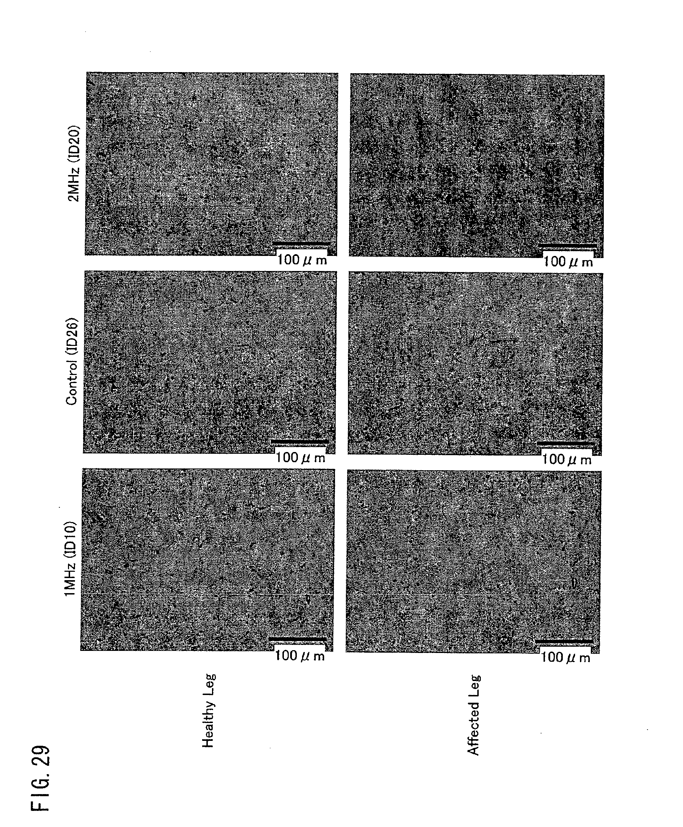

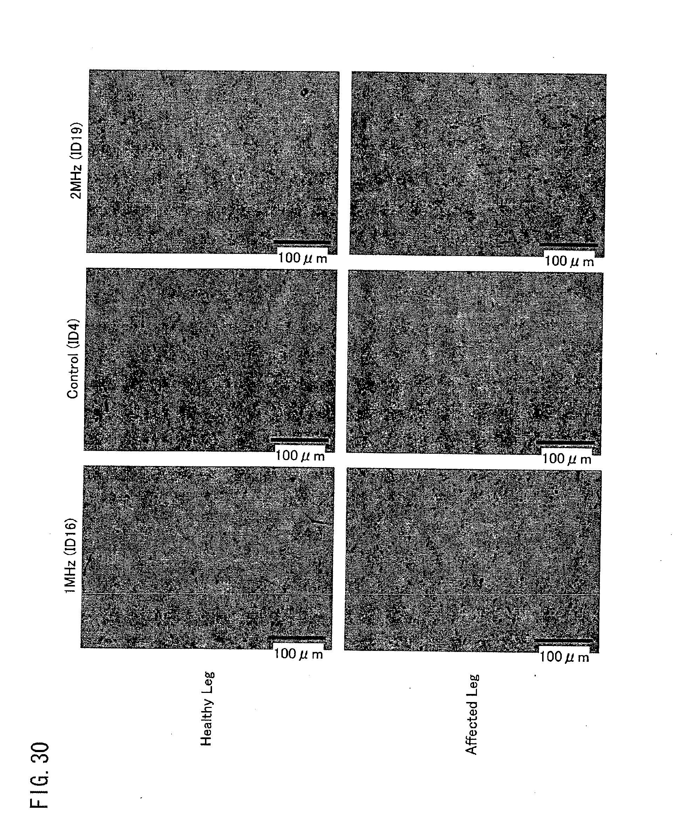

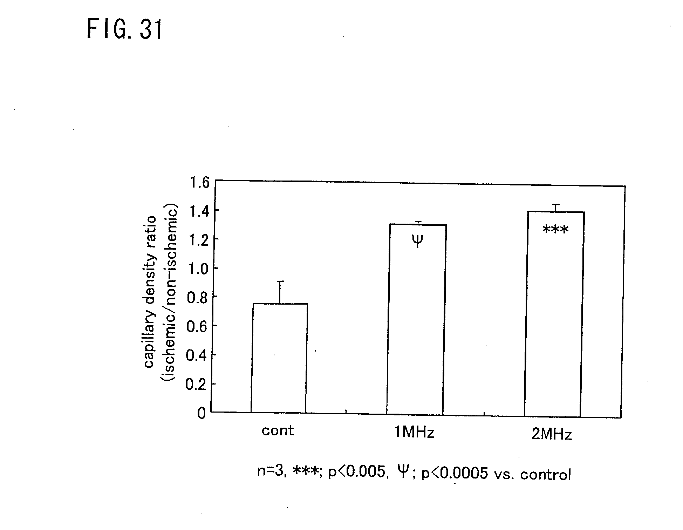

Figs. 28 through 30 are micrographs taken by an inverted optical microscope (OLYMPUS IX81) with respect to the tissue sections subjected to immunohistochemistry staining as in the same manner using the CD31 antibodies. As in the case with the sections obtained by alkaline phosphatase staining, spots positively reacted to CD31 were counted, and a capillary density ratio of the affected leg to the healthy leg was shown in a graph (Fig. 31).

As shown in Figs. 28 through 30, a large number of CD31 positive reactions were confirmed in the affected legs of the individuals ID15,ID10, and ID16 that were irradiated with ultrasonic waves at a frequency of 1 MHz and the individuals ID21, ID20, and ID19 that were irradiated with ultrasonic waves at a frequency of 2 MHz. On the other hand, a small number of CD31 positive reactions were confirmed in the affected legs of the control individuals ID7, ID26, and ID4 which were not irradiated with ultrasonic waves. As shown in Fig. 31, a capillary density ratio, which is obtained based on the number of spots positively reacted to CD31, of the ultrasonic irradiation group was higher than that of the control group that was not irradiated with ultrasonic waves.

### Industrial Applicability

A device for modulating PGC-1α expression according to the present invention enables modulation of VEGF expression level by modulating PGC-1α expression in a living body, thus allowing promotion of angiogenesis. Therefore, the device for modulating PGC-1α expression according to the present invention is applicable to treating devices for ischemic diseases like arteriosclerosis obliterans (ASO) and Burger's disease.

### Reference Signs List

- 1, 1a: PGC-1 expression-modulating device
- 10, 10a: Main unit
- 11: User interface
- 12: Controller (controlling means)
- 13: Power source section
- 14: Ultrasonic wave transmitting section (ultrasonic irradiation means)
- 15: Selector switch
- 20, 20a: Probe
- 21: Transducer (ultrasonic emission terminal)

## Claims

1. A non-invasive device (1) for modulation of PGC-1 expression in a target tissue, the device comprising a controller (12) and ultrasonic irradiation means (14), the device having specific parameters of ultrasonic irradiation appropriate for modulating PGC-1 expression,
wherein the specific parameters of ultrasonic irradiation are such that the frequency of ultrasonic waves is in a range from 0.5 MHz to 3 MHz ± 10 %, the irradiation time is in a range from 20 minutes to 40 minutes ± 10 % per day, the duty ratio is 10% ± 10 % or 20% ± 10 % and PRF is 1 kHz ± 10 % or 100 kHz ± 10 %; and
wherein the controller (12) is configured to get feedback on an expression level of PGC-1 or VEGF measured at a site subjected to ultrasonic irradiation, and control ultrasonic wave output from the ultrasonic irradiation means (14) in accordance with the feedback.

2. The device (1) according to claim 1, wherein
the ultrasonic irradiation means (14) further includes at least one ultrasonic emission terminal (141) that non-invasively contacts the subject via a medium and emits the ultrasonic waves for irradiation of the subject.

3. The device (1) according to claim 1 or 2, comprising:
controlling means (12) for controlling the ultrasonic irradiation means (14) so that a plurality of the ultrasonic emission terminals (141) are sequentially driven by time division to emit the ultrasonic waves.

4. The device according to any one of claims 1 through 3, configured to treat ischemic disease.

## Patentansprüche

1. Niichtinvasive Vorrichtung (1) zur Modulation der PGC-1-Expression in einem Zielgewebe, wobei die Vorrichtung eine Steuerung (12) und eine Ultraschallbestrahlungseinrichtung (14) umfasst, wobei die Vorrichtung spezifische, zur Modulation der PGC-1-Expression geeignete Ultraschallbestrahlungsparameter aufweist,
wobei die spezifischen Ultraschallbestrahlungsparameter so sind, dass die Ultraschallwellenfrequenz in einem Bereich von 0,5 MHz bis 3 MHz ± 10 % liegt, die Bestrahlungszeit in einem Bereich von 20 Minuten bis 40 Minuten ± 10 % pro Tag liegt, das Tastverhältnis 10 % ± 10 % oder 20% ± 10 % beträgt und Impulsfolgefrequenz 1 kHz ± 10 % oder 100 kHz ± 10 % beträgt; und
wobei die Steuerung (12) gestaltet ist, um eine Rückmeldung zu einem PGC-1- oder VEGF-Expressionsniveau, das an einem einer Ultraschallbestrahlung ausgesetzten Ort gemessen wurde, zu erhalten und einen Ultraschallwellenausgang der Ultraschallbestrahlungseinrichtung (14) in Übereinstimmung mit der Rückmeldung zu steuern.

2. Vorrichtung (1) nach Anspruch 1, wobei
die Ultraschallbestrahlungseinrichtung (14) ferner zumindest ein Ultraschallemissionsterminal (141) aufweist, das mit dem Subjekt in nichtinvasiver Weise über ein Medium in Kontakt steht und die Ultraschallwellen zur Bestrahlung des Subjekts emittiert.

3. Vorrichtung (1) nach Anspruch 1 oder 2, umfassend:
eine Steuerungseinrichtung (12) zur Steuerung der Ultraschallbestrahlungseinrichtung (14), sodass eine Vielzahl der Ultraschallemissionsterminals (141) sequenziell durch Zeitdivision betrieben wird, um die Ultraschallwellen zu emittieren.

4. Vorrichtung nach einem der Ansprüche 1 bis 3, die gestaltet ist, ischämische Erkrankungen zu behandeln.

## Revendications

1. Dispositif non effractif (1) pour la modulation de l'expression de PGC-1 dans un tissu cible, le dispositif comprenant un dispositif de commande (12) et des moyens d'irradiation ultrasonique (14), le dispositif possédant des paramètres spécifiques d'irradiation ultrasonique appropriés pour moduler l'expression de PGC-1,
dans lequel les paramètres spécifiques d'irradiation ultrasonique sont tels que la fréquence d'ondes ultrasonores soit dans une plage de 0,5 MHz à 3 MHz ± 10 %, le temps d'irradiation soit dans une plage de 20 minutes à 40 minutes ± 10 % par jour, le facteur de marche soit 10 % ± 10 % ou 20% ± 10 % et PRF soit 1 kHz ± 10 % ou 100 kHz ± 10 %, et
dans lequel le dispositif de commande (12) est configuré pour obtenir une rétroaction sur un niveau d'expression de PGC-1 ou VEGF mesuré à un site soumis à un rayonnement ultrasonique, et commander la sortie d'ondes ultrasonores à partir des moyens d'irradiation ultrasonique (14) conformément à la rétroaction.

2. Dispositif (1) selon la revendication 1, dans lequel les moyens d'irradiation ultrasonique (14) comprennent en outre au moins une borne d'émission ultrasonique (141) qui entre en contact de façon non effractive avec le sujet par l'intermédiaire d'un milieu de contact et émet les ondes ultrasonores pour l'irradiation du sujet.

3. Dispositif (1) selon la revendication 1 ou 2, comprenant :
des moyens de commande (12) pour commander les moyens d'irradiation ultrasonique (14) pour qu'une pluralité des bornes d'émission ultrasonique (141) soient excitées séquentiellement par répartition dans le temps pour émettre les ondes ultrasonores.

4. Dispositif (1) selon une quelconque des revendications 1 à 3, configuré pour traiter une maladie ischémique.
